# EUROPEAN PATENT APPLICATION

(11) **EP 4 365 865 A2**
(43) Date of publication of application: **08.05.2024**
(21) Application number: 23207634.9
(22) Date of filing: 03.11.2023
(51) Int. Cl.: G08B 21/02, G08B 21/22, G08B 25/01

(54) **METHOD AND APPARATUS FOR RECEIVING ALERTS AND HEALTH DATA OF PASSENGERS**

(30) Priority: 07.11.2022 US 202217981970
(71) Applicant: Volvo Car Corporation, 405 31 Göteborg (SE)
(72) Inventor: Barcia, Peter, 405 31 Göteborg (SE); Raimondi, Anthony, 405 31 Göteborg (SE); Roselli, Ronald J., 405 31 Göteborg (SE); Caroncino, Kyle, 405 31 Göteborg (SE); Boesch, Derek, 405 31 Göteborg (SE); Spiritoso, Giovanni, 405 31 Göteborg (SE)
(74) Representative: Nederlandsch Octrooibureau

(57) **Abstract**

An embodiment relates to a system (400), wherein a system (400) is configured to be a component of a vehicle (401). The system (400) comprises a sensor (404), a camera (406), a connector (410), a communication module (420), and a processor (402). The processor (402) is configured to detect a vehicle seat in the vehicle via the connector (421), establish a secure connection with the vehicle seat through the connector (422), receive a first data of the vehicle from the sensor (424), receive a second data from the camera (426), process the first data and the second data (428), notify a condition of an occupant through the communication module (430) and notify a condition of the vehicle seat through the communication module (450).

## Description

### FIELD OF THE INVENTION

The present disclosure relates generally to the vehicle communication field. More specifically, the present disclosure relates to systems and methods for monitoring and detection of a health emergency of occupants in a vehicle by detecting a vehicle seat and establishing secure communication with the vehicle seat.

### BACKGROUND

The connected vehicle infrastructure provides a platform for collecting and utilizing much vehicle data that is used advantageously in a wide variety of applications. Such applications are not limited to vehicle control and infotainment, but may also involve vehicle safety. One such safety issue is the availability of situational awareness.

The problem in today's cars is that there is no way to know if a child, rear facing, or an occupant seated in a vehicle is having a medical issue, such as choking, high fever, and low vital signs.

Therefore, there is a need for a system and method that may monitor and detect a health issue in real-time and provide suggestions when an emergency, for example a health emergency, is detected.

### SUMMARY

The following presents a summary to provide a basic understanding of one or more embodiments described herein. This summary is not intended to identify key or critical elements or delineate any scope of the different embodiments and/or any scope of the claims. The sole purpose of the summary is to present some concepts in a simplified form as a prelude to the more detailed description presented herein.

An embodiment relates to a system, wherein a system is configured to be a component of a vehicle. The system comprises a sensor, a camera, a connector, a communication module, and a processor. The processor is configured to detect a vehicle seat in the vehicle via the connector, establish a secure connection with the vehicle seat through the connector, receive a first data of the vehicle from the sensor, receive a second data from the camera, process the first data and the second data, and notify a condition of the vehicle seat through the communication module.

An embodiment relates to a method comprising detecting a vehicle seat in the vehicle via the connector, establishing a secure connection with the vehicle seat through the connector, receiving a first data of the vehicle from the sensor, receiving a second data from the camera, processing the first data and the second data, and notifying a condition of the vehicle seat through the communication module.

An embodiment relates to a non-transitory computer storage medium storing a sequence of instructions, which when executed by a processor, causes detecting a vehicle seat in the vehicle via the connector, establishing a secure connection with the vehicle seat through the connector, receiving a first data of the vehicle from the sensor, receiving a second data from the camera, processing the first data and the second data, notifying a condition of the vehicle seat through the communication module.

An embodiment relates to a system, wherein a system is configured to be a component of a vehicle. The system comprises a sensor, a camera, a connector, a communication module, and a processor. The processor is configured to detect a vehicle seat in the vehicle via the connector, establish a secure connection with the vehicle seat through the connector, receive a first data of the vehicle from the sensor, receive a second data from the camera, process the first data and the second data, and notify a condition of an occupant through the communication module.

An embodiment relates to a method comprising detecting a vehicle seat in the vehicle via the connector, establishing a secure connection with the vehicle seat through the connector, receiving a first data of the vehicle from the sensor, receiving a second data from the camera, processing the first data and the second data, and notifying a condition of an occupant through the communication module.

An embodiment relates to a non-transitory computer storage medium storing a sequence of instructions, which when executed by a processor, causes detecting a vehicle seat in the vehicle via the connector, establishing a secure connection with the vehicle seat through the connector, receiving a first data of the vehicle from the sensor, receiving a second data from the camera, processing the first data and the second data, and notifying a condition of an occupant through the communication module.

An embodiment relates to a system, configured to: receive a software application installation package over a computer network, and install the software application onto a computing hardware associated with a vehicle. the software application comprises set of instructions executable by the computing hardware and stored in a non-transitory storage medium that, when executed, cause the computing hardware to implement operations comprising detecting a vehicle seat in the vehicle via a connector, establishing a secure connection with the vehicle seat through the connector, receiving a first data of the vehicle from a sensor, receiving a second data from a camera, processing the first data and the second data and notifying a condition of an occupant through a communication module.

An embodiment relates to system, configured to receive a software application installation package over a computer network, and install the software application onto a computing hardware associated with a vehicle. The software application comprises set of instructions executable by the computing hardware and stored in a non-transitory storage medium that, when executed, cause the computing hardware to implement operations comprising detecting a vehicle seat in the vehicle via a connector, establishing a secure connection with the vehicle seat through the connector, receiving a first data of the vehicle from a sensor, receiving a second data from a camera, processing the first data and the second data, and notifying a condition of the vehicle seat through a communication module.

### BRIEF DESCRIPTION OF THE FIGURES

These and other aspects of the present invention will now be described in more detail, with reference to the appended drawings showing exemplary embodiments of the present invention, in which:
FIG. 1 shows a block diagram of various components of a system to detect a vehicle seat and health risk inside the vehicle in one embodiment.
FIG. 2A shows a block diagram for a system to detect a vehicle seat in one embodiment.
FIG. 2B shows a flowchart for a method to detect a vehicle seat in one embodiment.
FIG. 3 shows a flowchart to detect a vehicle seat and exchange data by establishing a secure connection.
FIG. 4A shows a block diagram of a system to detect a health risk inside a vehicle according to one embodiment.
FIG. 4B shows a block diagram of a system to detect a health risk inside a vehicle according to another embodiment.
FIG. 5A shows a flowchart for detecting or predicting medical emergency from the sensor data according to an embodiment.
FIG. 5B shows a method for receiving alerts and health data of passengers according to an embodiment.
FIG. 5C shows a method for receiving alerts and condition of vehicle seat according to an embodiment.
FIG. 6A shows a structure of the neural network / machine learning model with a feedback loop.
FIG. 6B shows a structure of the neural network / machine learning model with reinforcement learning.
FIG. 6C shows an example block diagram for detecting a health emergency using a machine learning model.
FIG. 7 shows an example block diagram for detecting a health emergency using a machine learning model.
FIG. 8A shows a block diagram of the cyber security module in view of the system and server.
FIG. 8B shows an embodiment of the cyber security module.
FIG. 8C shows another embodiment of the cyber security module.
FIG. 9A shows the system with a software code to receive alerts and health data of passengers according to an embodiment.
FIG. 9B shows the system with a software code to receive alerts and condition of vehicle seat according to an embodiment.
FIG. 10A shows the system with a non-transitory storage medium with the software code to receive alerts and health data of passengers according to an embodiment.
FIG. 10B shows the system with a non-transitory storage medium with the software code to receive alerts and condition of vehicle seat according to an embodiment.

### DETAILED DESCRIPTION

For simplicity and clarity of illustration, the figures illustrate the general manner of construction. The description and figures may omit the descriptions and details of well-known features and techniques to avoid unnecessarily obscuring the present disclosure. The figures exaggerate the dimensions of some of the elements relative to other elements to help improve understanding of embodiments of the present disclosure. The same reference numeral in different figures denotes the same elements.

Although the herein detailed description contains many specifics for the purpose of illustration, a person of ordinary skill in the art will appreciate that many variations and alterations to the details are considered to be included herein.

Accordingly, the embodiments herein are without any loss of generality to, and without imposing limitations upon, any claims set forth. The terminology used herein is for the purpose of describing particular embodiments only and is not limiting. Unless defined otherwise, all technical and scientific terms used herein have the same meaning as commonly understood by one with ordinary skill in the art to which this disclosure belongs.

As used herein, the articles "a" and "an" used herein refer to one or to more than one (i.e., to at least one) of the grammatical object of the article. By way of example, "an element" means one element or more than one element. Moreover, usage of articles "a" and "an" in the subject specification and annexed drawings construe to mean "one or more" unless specified otherwise or clear from context to mean a singular form.

As used herein, the terms "example" and/or "exemplary" mean serving as an example, instance, or illustration. For the avoidance of doubt, such examples do not limit the herein described subject matter. In addition, any aspect or design described herein as an "example" and/or "exemplary" is not necessarily preferred or advantageous over other aspects or designs, nor does it preclude equivalent exemplary structures and techniques known to those of ordinary skill in the art.

As used herein, the terms "first," "second," "third," and the like in the description and in the claims, if any, distinguish between similar elements and do not necessarily describe a particular sequence or chronological order. The terms are interchangeable under appropriate circumstances such that the embodiments herein are, for example, capable of operation in sequences other than those illustrated or otherwise described herein. Furthermore, the terms "include," "have," and any variations thereof, cover a non-exclusive inclusion such that a process, method, system, article, device, or apparatus that comprises a list of elements is not necessarily limiting to those elements, but may include other elements not expressly listed or inherent to such process, method, system, article, device, or apparatus.

As used herein, the terms "left," "right," "front," "back," "top," "bottom," "over," "under" and the like in the description and in the claims, if any, are for descriptive purposes and not necessarily for describing permanent relative positions. The terms so used are interchangeable under appropriate circumstances such that the embodiments of the apparatus, methods, and/or articles of manufacture described herein are, for example, capable of operation in other orientations than those illustrated or otherwise described herein.

No element act, or instruction used herein is critical or essential unless explicitly described as such. Furthermore, the term "set" includes items (e.g., related items, unrelated items, a combination of related items and unrelated items, etc.) and may be interchangeable with "one or more." Where only one item is intended, the term "one" or similar language is used. Also, the terms "has," "have," "having," or the like are open-ended terms. Further, the phrase "based on" means "based, at least in part, on" unless explicitly stated otherwise.

As used herein, the terms "system," "device," "unit," and/or "module" refer to a different component, component portion, or component of the various levels of the order. However, other expressions that achieve the same purpose may replace the terms.

As used herein, the terms "couple," "coupled," "couples," "coupling," and the like refer to connecting two or more elements mechanically, electrically, and/or otherwise. Two or more electrical elements may be electrically coupled together, but not mechanically or otherwise coupled together. Coupling may be for any length of time, e.g., permanent, or semi-permanent or only for an instant. "Electrical coupling" includes electrical coupling of all types. The absence of the word "removably," "removable," and the like, near the word "coupled" and the like does not mean that the coupling, etc. in question is or is not removable.

As used herein, the term "or" means an inclusive "or" rather than an exclusive "or." That is, unless specified otherwise, or clear from context, "X employs A or B" means any of the natural inclusive permutations. That is, if X employs A; X employs B; or X employs both A and B, then "X employs A or B" is satisfied under any of the foregoing instances.

As used herein, two or more elements or modules are "integral" or "integrated" if they operate functionally together. Two or more elements are "non-integral" if each element can operate functionally independently.

As used herein, the term "real-time" refers to operations conducted as soon as practically possible upon occurrence of a triggering event. A triggering event can include receipt of data necessary to execute a task or to otherwise process information. Because of delays inherent in transmission and/or in computing speeds, the term "real-time" encompasses operations that occur in "near" real-time or somewhat delayed from a triggering event. In a number of embodiments, "real-time" can mean real-time less a time delay for processing (e.g., determining) and/or transmitting data. The particular time delay can vary depending on the type and/or amount of the data, the processing speeds of the hardware, the transmission capability of the communication hardware, the transmission distance, etc. However, in many embodiments, the time delay can be less than approximately one second, two seconds, five seconds, or ten seconds.

As used herein, the term "approximately" can mean within a specified or unspecified range of the specified or unspecified stated value. In some embodiments, "approximately" can mean within plus or minus ten percent of the stated value. In other embodiments, "approximately" can mean within plus or minus five percent of the stated value. In further embodiments, "approximately" can mean within plus or minus three percent of the stated value. In yet other embodiments, "approximately" can mean within plus or minus one percent of the stated value.

Other specific forms may embody the present invention without departing from its spirit or characteristics. The described embodiments are in all respects illustrative and not restrictive. Therefore, the appended claims rather than the description herein indicate the scope of the invention. All variations which come within the meaning and range of equivalency of the claims are within their scope.

As used herein, the term "component" broadly construes hardware, firmware, and/or a combination of hardware, firmware, and software.

Digital electronic circuitry, or in computer software, firmware, or hardware, including the structures disclosed in this specification and their structural equivalents, or in combinations of one or more of them may realize the implementations and all of the functional operations described in this specification. Implementations may be as one or more computer program products i.e., one or more modules of computer program instructions encoded on a computer-readable medium for execution by, or to control the operation of, data processing apparatus. The computer-readable medium may be a machine-readable storage device, a machine-readable storage substrate, a memory device, a composition of matter affecting a machine-readable propagated signal, or a combination of one or more of them. The term "computing system" encompasses all apparatus, devices, and machines for processing data, including by way of example, a programmable processor, a computer, or multiple processors or computers. The apparatus may include, in addition to hardware, code that creates an execution environment for the computer program in question, e.g., code that constitutes processor firmware, a protocol stack, a database management system, an operating system, or a combination of one or more of them. A propagated signal is an artificially generated signal (e.g., a machine-generated electrical, optical, or electromagnetic signal) that encodes information for transmission to a suitable receiver apparatus.

The actual specialized control hardware or software code used to implement these systems and/or methods is not limiting to the implementations. Thus, any software and any hardware can implement the systems and/or methods based on the description herein without reference to specific software code.

A computer program (also known as a program, software, software application, script, or code) is written in any appropriate form of programming language, including compiled or interpreted languages. Any appropriate form, including a standalone program or a module, component, subroutine, or other unit suitable for use in a computing environment may deploy it. A computer program does not necessarily correspond to a file in a file system. A program may be stored in a portion of a file that holds other programs or data (e.g., one or more scripts stored in a markup language document), in a single file dedicated to the program in question, or in multiple coordinated files (e.g., files that store one or more modules, sub programs, or portions of code). A computer program may execute on one computer or on multiple computers that are located at one site or distributed across multiple sites and interconnected by a communication network.

One or more programmable processors, executing one or more computer programs to perform functions by operating on input data and generating output, perform the processes and logic flows described in this specification. The processes and logic flows may also be performed by, and apparatus may also be implemented as, special purpose logic circuitry, for example, without limitation, a Field Programmable Gate Array (FPGA), an Application Specific Integrated Circuit (ASIC), Application Specific Standard Products (ASSPs), System-On-a-Chip (SOC) systems, Complex Programmable Logic Devices (CPLDs), etc.

Processors suitable for the execution of a computer program include, by way of example, both general and special purpose microprocessors, and any one or more processors of any appropriate kind of a digital computer. A processor will receive instructions and data from a read-only memory or a random-access memory or both. Elements of a computer can include a processor for performing instructions and one or more memory devices for storing instructions and data. A computer will also include, or is operatively coupled to receive data, transfer data or both, to/from one or more mass storage devices for storing data e.g., magnetic disks, magneto optical disks, optical disks, or solid-state disks. However, a computer need not have such devices. Moreover, another device, e.g., a mobile telephone, a personal digital assistant (PDA), a mobile audio player, a Global Positioning System (GPS) receiver, etc. may embed a computer. Computer-readable media suitable for storing computer program instructions and data include all forms of non-volatile memory, media and memory devices, including, by way of example, semiconductor memory devices (e.g., Erasable Programmable Read-Only Memory (EPROM), Electronically Erasable Programmable Read-Only Memory (EEPROM), and flash memory devices), magnetic disks (e.g., internal hard disks or removable disks), magneto optical disks (e.g. Compact Disc Read-Only Memory (CD ROM) disks, Digital Versatile Disk-Read-Only Memory (DVD-ROM) disks) and solid-state disks. Special purpose logic circuitry may supplement or incorporate the processor and the memory.

To provide for interaction with a user, a computer may have a display device, e.g., a Cathode Ray Tube (CRT) or Liquid Crystal Display (LCD) monitor, for displaying information to the user, and a keyboard and a pointing device, e.g., a mouse or a trackball, by which the user may provide input to the computer. Other kinds of devices provide for interaction with a user as well. For example, feedback to the user may be any appropriate form of sensory feedback, e.g., visual feedback, auditory feedback, or tactile feedback; and a computer may receive input from the user in any appropriate form, including acoustic, speech, or tactile input.

A computing system that includes a back-end component, e.g., a data server, or that includes a middleware component, e.g., an application server, or that includes a front-end component, e.g., a client computer having a graphical user interface or a Web browser through which a user may interact with an implementation, or any appropriate combination of one or more such back-end, middleware, or front-end components, may realize implementations described herein. Any appropriate form or medium of digital data communication, e.g., a communication network may interconnect the components of the system. Examples of communication networks include a Local Area Network (LAN) and a Wide Area Network (WAN), e.g., Intranet and Internet.

The computing system may include clients and servers. A client and server are remote from each other and typically interact through a communication network. The relationship of the client and server arises by virtue of computer programs running on the respective computers and having a client-server relationship to each other.

Embodiments of the present invention may comprise or utilize a special purpose or general purpose computer including computer hardware. Embodiments within the scope of the present invention may also include physical and other computer-readable media for carrying or storing computer-executable instructions and/or data structures. Such computer-readable media can be any media accessible by a general purpose or special purpose computer system. Computer-readable media that store computer-executable instructions are physical storage media. Computer-readable media that carry computer-executable instructions are transmission media. Thus, by way of example and not limitation, embodiments of the invention can comprise at least two distinct kinds of computer-readable media: physical computer-readable storage media and transmission computer-readable media.

Although the present embodiments described herein are with reference to specific example embodiments it will be evident that various modifications and changes may be made to these embodiments without departing from the broader spirit and scope of the various embodiments. For example, hardware circuitry (e.g., Complementary Metal Oxide Semiconductor (CMOS) based logic circuitry), firmware, software (e.g., embodied in a non-transitory machine-readable medium), or any combination of hardware, firmware, and software may enable and operate the various devices, units, and modules described herein. For example, transistors, logic gates, and electrical circuits (e.g., Application Specific Integrated Circuit (ASIC) and/or Digital Signal Processor (DSP) circuit) may embody the various electrical structures and methods.

In addition, a non-transitory machine-readable medium and/or a system may embody the various operations, processes, and methods disclosed herein. Accordingly, the specification and drawings are illustrative rather than restrictive.

Physical computer-readable storage media includes RAM, ROM, EEPROM, CD-ROM or other optical disk storage (such as CDs, DVDs, etc.), magnetic disk storage or other magnetic storage devices, solid-state disks or any other medium. They store desired program code in the form of computer-executable instructions or data structures which can be accessed by a general purpose or special purpose computer.

As used herein, the term "network" refers to one or more data links that enable the transport of electronic data between computer systems and/or modules and/or other electronic devices. When a network or another communications connection (either hardwired, wireless, or a combination of hardwired or wireless) transfers or provides information to a computer, the computer properly views the connection as a transmission medium. A general purpose or special purpose computer access transmission media that can include a network and / or data links which carry desired program code in the form of computer-executable instructions or data structures. The scope of computer-readable media includes combinations of the above, that enable the transport of electronic data between computer systems and/or modules and/or other electronic devices.

Further, upon reaching various computer system components, program code in the form of computer-executable instructions or data structures can be transferred automatically from transmission computer-readable media to physical computer-readable storage media (or vice versa). For example, computer-executable instructions or data structures received over a network or data link can be buffered in RAM within a Network Interface Module (NIC), and then eventually transferred to computer system RAM and/or to less volatile computer-readable physical storage media at a computer system. Thus, computer system components that also (or even primarily) utilize transmission media may include computer-readable physical storage media.

Computer-executable instructions comprise, for example, instructions and data which cause a general purpose computer, special purpose computer, or special purpose processing device to perform a certain function or group of functions. The computer-executable instructions may be, for example, binary, intermediate format instructions such as assembly language, or even source code. Although the subject matter herein described is in a language specific to structural features and/or methodological acts, the described features or acts described do not limit the subject matter defined in the claims. Rather, the herein described features and acts are example forms of implementing the claims.

While this specification contains many specifics, these do not construe as limitations on the scope of the disclosure or of the claims, but as descriptions of features specific to particular implementations. A single implementation may implement certain features described in this specification in the context of separate implementations. Conversely, multiple implementations separately or in any suitable sub-combination may implement various features described herein in the context of a single implementation. Moreover, although features described herein as acting in certain combinations and even initially claimed as such, one or more features from a claimed combination may in some cases be excised from the combination, and the claimed combination may be directed to a sub-combination or variation of a sub-combination.

Similarly, while operations depicted herein in the drawings in a particular order to achieve desired results, this should not be understood as requiring that such operations be performed in the particular order shown or in sequential order or that all illustrated operations be performed, to achieve desirable results. In certain circumstances, multitasking and parallel processing may be advantageous. Moreover, the separation of various system components in the implementations should not be understood as requiring such separation in all implementations, and it should be understood that the described program components and systems may be integrated together in a single software product or packaged into multiple software products.

Even though particular combinations of features are recited in the claims and/or disclosed in the specification, these combinations are not intended to limit the disclosure of possible implementations. Other implementations are within the scope of the claims. For example, the actions recited in the claims may be performed in a different order and still achieve desirable results. In fact, many of these features may be combined in ways not specifically recited in the claims and/or disclosed in the specification. Although each dependent claim listed below may directly depend on only one claim, the disclosure of possible implementations includes each dependent claim in combination with every other claim in the claim set.

Further, a computer system including one or more processors and computer-readable media such as computer memory may practice the methods. In particular, one or more processors execute computer-executable instructions, stored in the computer memory, to perform various functions such as the acts recited in the embodiments.

Those skilled in the art will appreciate that the invention may be practiced in network computing environments with many types of computer system configurations including personal computers, desktop computers, laptop computers, message processors, hand-held devices, multiprocessor systems, microprocessor-based or programmable consumer electronics, network PCs, minicomputers, mainframe computers, mobile telephones, PDAs, pagers, routers, switches, etc. Distributed system environments where local and remote computer systems, which are linked (either by hardwired data links, wireless data links, or by a combination of hardwired and wireless data links) through a network, both perform tasks may also practice the invention. In a distributed system environment, program modules may be located in both local and remote memory storage devices.

The embodiments described herein can be directed to one or more of a system, a method, an apparatus, and/or a computer program product at any possible technical detail level of integration. The computer program product can include a computer readable storage medium (or media) having computer readable program instructions thereon for causing a processor to carry out aspects of the one or more embodiments described herein. The computer readable storage medium can be a tangible device that can retain and store instructions for use by an instruction execution device. For example, the computer readable storage medium can be, but is not limited to, an electronic storage device, a magnetic storage device, an optical storage device, an electromagnetic storage device, a superconducting storage device, and/or any suitable combination of the foregoing. A non-exhaustive list of more specific examples of the computer readable storage medium can also include the following: a portable computer diskette, a hard disk, a random access memory (RAM), a read-only memory (ROM), an erasable programmable read-only memory (EPROM or Flash memory), a static random access memory (SRAM), a portable compact disc read-only memory (CD-ROM), a digital versatile disk (DVD), a memory stick, a floppy disk, a mechanically encoded device such as punch-cards or raised structures in a groove having instructions recorded thereon and/or any suitable combination of the foregoing. A computer readable storage medium, as used herein, does not construe transitory signals per se, such as radio waves and/or other freely propagating electromagnetic waves, electromagnetic waves propagating through a waveguide and/or other transmission media (e.g., light pulses passing through a fiber-optic cable), and/or electrical signals transmitted through a wire.

Computer readable program instructions described herein are downloadable to respective computing/processing devices from a computer readable storage medium and/or to an external computer or external storage device via a network, for example, the Internet, a local area network, a wide area network and/or a wireless network. The network can comprise copper transmission cables, optical transmission fibers, wireless transmission, routers, firewalls, switches, gateway computers, and/or edge servers. A network adapter card or network interface in each computing/processing device receives computer readable program instructions from the network and forwards the computer readable program instructions for storage in a computer readable storage medium within the respective computing/processing device. Computer readable program instructions for carrying out operations of the one or more embodiments described herein can be assembler instructions, instruction-set-architecture (ISA) instructions, machine instructions, machine dependent instructions, microcode, firmware instructions, state-setting data, configuration data for integrated circuitry, and/or source code and/or object code written in any combination of one or more programming languages, including an object oriented programming language such as Smalltalk, C++ or the like, and/or procedural programming languages, such as the "C" programming language and/or similar programming languages. The computer readable program instructions can execute entirely on a computer, partly on a computer, as a stand-alone software package, partly on a computer and/or partly on a remote computer or entirely on the remote computer and/or server. In the latter scenario, the remote computer can be connected to a computer through any type of network, including a local area network (LAN) and/or a wide area network (WAN), and/or the connection can be made to an external computer (for example, through the Internet using an Internet Service Provider). In one or more embodiments, electronic circuitry including, for example, programmable logic circuitry, field-programmable gate arrays (FPGA), and/or programmable logic arrays (PLA) can execute the computer readable program instructions by utilizing state information of the computer readable program instructions to personalize the electronic circuitry, in order to perform aspects of the one or more embodiments described herein.

Aspects of the one or more embodiments described herein are described with reference to flowchart illustrations and/or block diagrams of methods, apparatus (systems), and computer program products according to one or more embodiments described herein. Each block of the flowchart illustrations and/or block diagrams, and combinations of blocks in the flowchart illustrations and/or block diagrams, can be implemented by computer readable program instructions. These computer readable program instructions can be provided to a processor of a general purpose computer, special purpose computer and/or other programmable data processing apparatus to produce a machine, such that the instructions, which execute via the processor of the computer or other programmable data processing apparatus, can create means for implementing the functions/acts specified in the flowchart and/or block diagram block or blocks. These computer readable program instructions can also be stored in a computer readable storage medium that can direct a computer, a programmable data processing apparatus and/or other devices to function in a particular manner, such that the computer readable storage medium having instructions stored therein can comprise an article of manufacture including instructions which can implement aspects of the function/act specified in the flowchart and/or block diagram block or blocks. The computer readable program instructions can also be loaded onto a computer, other programmable data processing apparatus and/or other device to cause a series of operational acts to be performed on the computer, other programmable apparatus and/or other device to produce a computer implemented process, such that the instructions which execute on the computer, other programmable apparatus and/or other device implement the functions/acts specified in the flowchart and/or block diagram block or blocks.

The flowcharts and block diagrams in the figures illustrate the architecture, functionality and/or operation of possible implementations of systems, computer-implementable methods and/or computer program products according to one or more embodiments described herein. In this regard, each block in the flowchart or block diagrams can represent a module, segment and/or portion of instructions, which comprises one or more executable instructions for implementing the specified logical function(s). In one or more alternative implementations, the functions noted in the blocks can occur out of the order noted in the Figures. For example, two blocks shown in succession can be executed substantially concurrently, and/or the blocks can sometimes be executed in the reverse order, depending upon the functionality involved. It will also be noted that each block of the block diagrams and/or flowchart illustration, and/or combinations of blocks in the block diagrams and/or flowchart illustration, can be implemented by special purpose hardware-based systems that can perform the specified functions and/or acts and/or carry out one or more combinations of special purpose hardware and/or computer instructions.

While the subject matter described herein is in the general context of computer-executable instructions of a computer program product that runs on a computer and/or computers, those skilled in the art will recognize that the one or more embodiments herein also can be implemented in combination with one or more other program modules. Program modules include routines, programs, components, data structures, and/or the like that perform particular tasks and/or implement particular abstract data types. Moreover, other computer system configurations, including single-processor and/or multiprocessor computer systems, mini-computing devices, mainframe computers, as well as computers, hand-held computing devices (e.g., PDA, phone), microprocessor-based or programmable consumer and/or industrial electronics and/or the like can practice the herein described computer-implemented methods. Distributed computing environments, in which remote processing devices linked through a communications network perform tasks, can also practice the illustrated aspects. However, stand-alone computers can practice one or more, if not all aspects of the one or more embodiments described herein. In a distributed computing environment, program modules can be located in both local and remote memory storage devices.

As used in this application, the terms "component," "system," "platform," "interface," and/or the like, can refer to and/or can include a computer-related entity or an entity related to an operational machine with one or more specific functionalities. The entities described herein can be either hardware, a combination of hardware and software, software, or software in execution. For example, a component can be, but is not limited to being, a process running on a processor, a processor, an object, an executable, a thread of execution, a program and/or a computer. By way of illustration, both an application running on a server and the server can be a component. One or more components can reside within a process and/or thread of execution and a component can be localized on one computer and/or distributed between two or more computers. In another example, respective components can execute from various computer readable media having various data structures stored thereon. The components can communicate via local and/or remote processes such as in accordance with a signal having one or more data packets (e.g., data from one component interacting with another component in a local system, distributed system and/or across a network such as the Internet with other systems via the signal). As another example, a component can be an apparatus with specific functionality provided by mechanical parts operated by electric or electronic circuitry, which is operated by a software and/or firmware application executed by a processor. In such a case, the processor can be internal and/or external to the apparatus and can execute at least a part of the software and/or firmware application. As yet another example, a component can be an apparatus that provides specific functionality through electronic components without mechanical parts, where the electronic components can include a processor and/or other means to execute software and/or firmware that confers at least in part the functionality of the electronic components. In an aspect, a component can emulate an electronic component via a virtual machine, e.g., within a cloud computing system.

As it is employed in the subject specification, the term "processor" can refer to any computing processing unit and/or device comprising, but not limited to, single-core processors; single-processors with software multithread execution capability; multi-core processors; multi-core processors with software multithread execution capability; multi-core processors with hardware multithread technology; parallel platforms; and/or parallel platforms with distributed shared memory. Additionally, a processor can refer to an integrated circuit, an application specific integrated circuit (ASIC), a digital signal processor (DSP), a field programmable gate array (FPGA), a programmable logic controller (PLC), a complex programmable logic device (CPLD), a discrete gate or transistor logic, discrete hardware components, and/or any combination thereof designed to perform the functions described herein. Further, processors can exploit nano-scale architectures such as, but not limited to, molecular based transistors, switches and/or gates, in order to optimize space usage and/or to enhance performance of related equipment. A combination of computing processing units can implement a processor.

Herein, terms such as "store," "storage," "data store," data storage," "database," and any other information storage component relevant to operation and functionality of a component refer to "memory components," entities embodied in a "memory," or components comprising a memory. Memory and/or memory components described herein can be either volatile memory or nonvolatile memory or can include both volatile and nonvolatile memory. By way of illustration, and not limitation, nonvolatile memory can include read only memory (ROM), programmable ROM (PROM), electrically programmable ROM (EPROM), electrically erasable ROM (EEPROM), flash memory, and/or nonvolatile random access memory (RAM) (e.g., ferroelectric RAM (FeRAM). Volatile memory can include RAM, which can function as external cache memory, for example. By way of illustration and not limitation, RAM can be available in many forms such as synchronous RAM (SRAM), dynamic RAM (DRAM), synchronous DRAM (SDRAM), double data rate SDRAM (DDR SDRAM), enhanced SDRAM (ESDRAM), Synch link DRAM (SLDRAM), direct Rambus RAM (DRRAM), direct Rambus dynamic RAM (DRDRAM) and/or Rambus dynamic RAM (RDRAM). Additionally, the described memory components of systems and/or computer-implemented methods herein include, without being limited to including, these and/or any other suitable types of memory.

The embodiments described herein include mere examples of systems and computer-implemented methods. It is, of course, not possible to describe every conceivable combination of components and/or computer-implemented methods for purposes of describing the one or more embodiments, but one of ordinary skill in the art can recognize that many further combinations and/or permutations of the one or more embodiments are possible. Furthermore, to the extent that the terms "includes," "has," "possesses," and the like are used in the detailed description, claims, appendices and/or drawings such terms are intended to be inclusive in a manner similar to the term "comprising" as "comprising" is interpreted when employed as a transitional word in a claim.

The descriptions of the one or more embodiments are for purposes of illustration but are not exhaustive or limiting to the embodiments described herein. Many modifications and variations will be apparent to those of ordinary skill in the art without departing from the scope and spirit of the described embodiments. The terminology used herein best explains the principles of the embodiments, the practical application and/or technical improvement over technologies found in the marketplace, and/or to enable others of ordinary skill in the art to understand the embodiments described herein.

As used herein, the term "Cryptographic protocol" is also known as security protocol or encryption protocol. It is an abstract or concrete protocol that performs a security-related function and applies cryptographic methods often as sequences of cryptographic primitives. A protocol describes the use of the algorithms. A sufficiently detailed protocol includes details about data structures and representations, at which point it is used to implement multiple, interoperable versions of a program.

Cryptographic protocols are widely used for secure application-level data transport. A cryptographic protocol usually incorporates at least some of these aspects: key agreement or establishment, entity authentication, symmetric encryption, and message authentication material construction, secured application-level data transport, non-repudiation methods, secret sharing methods, and secure multi-party computation.

As used herein, the term "IoT" stands for Internet of Things which describes the network of physical objects "things" or objects that are embedded with sensors, software, and other technologies for the purpose of connecting and exchanging data with other devices and systems over the internet.

As used herein "Machine learning" refers to algorithms that give a computer the ability to learn without being explicitly programmed, including algorithms that learn from and make predictions about data. Machine learning algorithms include, but are not limited to, decision tree learning, artificial neural networks (ANN) (also referred to herein as a "neural net"), deep learning neural network, support vector machines, rules-based machine learning, random forest, etc. For the purposes of clarity, algorithms such as linear regression or logistic regression may also be used as part of a machine learning process. However, it is understood that using linear regression or another algorithm as part of a machine learning process is distinct from performing a statistical analysis such as regression with a spreadsheet program. The machine learning process may continually learn and adjust the classifier as new data becomes available and does not rely on explicit or rules-based programming. The ANN is featured with a feedback loop to adjust the system output dynamically as it learns from the new data as it becomes available. In machine learning, backpropagation and feedback loops are used to train the AI/ML model improving the model's accuracy and performance over time.

Statistical modeling relies on finding relationships between variables (e.g., mathematical equations) to predict an outcome.

As used herein, the term "Data mining" is a process used to turn raw data into useful information.

As used herein, the term "Data acquisition" is the process of sampling signals that measure real world physical conditions and converting the resulting samples into digital numeric values, and the computer manipulates the values. Data acquisition systems typically convert analog waveforms into digital values for processing. The components of data acquisition systems include sensors to convert physical parameters to electrical signals, signal conditioning circuitry to convert sensor signals into a form that is converted to digital values, and analog-to-digital converters to convert conditioned sensor signals to digital values. Stand-alone data acquisition systems are often called data loggers.

As used herein, the term "Dashboard" is a type of interface that visualizes particular Key Performance Indicators (KPIs) for a specific goal or process. It is based on data visualization and infographics.

As used herein, a "Database" is a collection of organized information so that it is easily accessed, managed, and updated. Computer databases typically contain aggregations of data records or files.

As used herein, the term "Data set" (or "Dataset") is a collection of data. In the case of tabular data, a data set corresponds to one or more database tables, where every column of a table represents a particular variable, and each row corresponds to a given record of the data set in question. The data set lists values for each of the variables, such as height and weight of an object, for each member of the data set. Each value is known as a datum. Data sets may also consist of a collection of documents or files.

As used herein, a "Sensor" is a device that measures physical input from its environment and converts it into data. A human or machine may interpret this data. Most sensors are electronic (The system converts the data into electronic data), but some are simpler, such as a glass thermometer, which presents visual data.

As used herein, the term "ODBC" stands for Open Database Connectivity and is a standard application programming interface (API) for accessing database management systems (DBMS). The designers of ODBC aimed to make it independent of database systems and operating systems. An application written using ODBC is ported to other platforms, both on the client and the server side, with few changes to the data access code.

The following terms and phrases, unless otherwise indicated, shall be understood to have the following meanings.

The term "vehicle" as used herein refers to a thing used for transporting people or goods. Automobiles, cars, trucks, buses etc. are examples of vehicles.

The term "electronic control unit" (ECU), also known as an "electronic control module" (ECM), is a system that controls one or more subsystems. An ECU may be installed in a car or other motor vehicle. It may refer to many ECUs, and can include but not limited to, Engine Control Module (ECM), Powertrain Control Module (PCM), Transmission Control Module (TCM), Brake Control Module (BCM) or Electronic Brake Control Module (EBCM), Central Control Module (CCM), Central Timing Module (CTM), General Electronic Module (GEM), Body Control Module (BCM), and Suspension Control Module (SCM). ECUs together are sometimes referred to collectively as the vehicles' computer or vehicles' central computer and may include separate computers. In an example, the electronic control unit can be embedded system in automotive electronics. In another example, the electronic control unit is wirelessly coupled with the automotive electronics.

The term "infotainment system" or "in-vehicle infotainment system" (IVI) as used herein refers to a combination of systems which are used to deliver entertainment and information. In an example, the information may be delivered to the driver and the passengers of a vehicle through audio/ video interfaces, control elements like touch screen displays, button panel, voice commands, and more. Some of the main components of an in-vehicle infotainment systems are integrated headunit, heads-up display, high-end Digital Signal Processors (DSPs), and Graphics Processing Units (GPUs) to support multiple displays, operating systems, Controller Area Network (CAN), Low-Voltage Differential Signaling (LVDS), and other network protocol support (as per the requirement), connectivity modules, automotive sensors integration, digital instrument cluster, etc.

The term "environment" or "surrounding" as used herein refers to surroundings and the space in which a vehicle is navigating. It refers to dynamic surroundings in which a vehicle is navigating which includes other vehicles, obstacles, pedestrians, lane boundaries, traffic signs and signals, speed limits, potholes, snow, water logging etc. The term "communication system" or "communication module" as used herein refers to a system which enables the information exchange between at least two points. The process of transmission and reception of information is called communication. The major elements of communication include, but are not limited to, a transmitter of information, channel or medium of communication and a receiver of information.

The term "communication system" or "communication module" as used herein refers to a system which enables the information exchange between two points. The process of transmission and reception of information is called communication. The major elements of communication include but are not limited to a transmitter of information, channel or medium of communication and a receiver of information.

The term "autonomous mode" as used herein refers to an operating mode which is independent and unsupervised.

The term "autonomous communication" as used herein comprises communication over a period with minimal supervision under different scenarios and is not solely or completely based on pre-coded scenarios or pre-coded rules or a predefined protocol. Autonomous communication, in general, happens in an independent and an unsupervised manner. The term "protocol unit" or "message protocol unit" as used herein defines the rules and sequencing of communication structure between various types of units.

The term "autonomous mode" as used herein refers to an operating mode which is independent and unsupervised.

The term "communication system" or "communication module" as used herein refers to a system which enables the information exchange between two points. The process of transmission and reception of information is called communication. The major elements of communication include but are not limited to a transmitter of information, channel or medium of communication and a receiver of information.

The term "connection" as used herein refers to a communication link. It refers to a communication channel that connects two or more devices for the purpose of data transmission. It may refer to a physical transmission medium such as a wire, or to a logical connection over a multiplexed medium such as a radio channel in telecommunications and computer networking. A channel is used for information transfer of, for example a digital bit stream, from one or several senders to one or several receivers. A channel has a certain capacity for transmitting information, often measured by its bandwidth in Hertz (Hz) or its data rate in bits per second. For example, a Vehicle-to-Vehicle (V2V) communication may wirelessly exchange information about the speed, location and heading of surrounding vehicles.

The term "communication" as used herein refers to the transmission of information and/or data from one point to another. Communication may be by means of electromagnetic waves. It is also a flow of information from one point, known as the source, to another, the receiver. Communication comprises one of the following: transmitting data, instructions, and information or a combination of data, instructions, and information. Communication happens between any two communication systems or communicating units. The term "in communication with" may refer to any coupling, connection, or interaction using electrical signals to exchange information or data, using any system, hardware, software, protocol, or format, regardless of whether the exchange occurs wirelessly or over a wired connection. The term communication includes systems that combine other more specific types of communication, such as V2I (Vehicle-to-Infrastructure), V2I (Vehicle-to-Infrastructure), V2N (Vehicle-to-Network), V2V (Vehicle-to-Vehicle), V2P (Vehicle-to-Pedestrian), V2D (Vehicle-to-Device) and V2G (Vehicle-to-Grid) and Vehicle-to-Everything (V2X) communication. V2X communication is the transmission of information from a vehicle to any entity that may affect the vehicle, and vice versa. The main motivations for developing V2X are occupant safety, road safety, traffic efficiency and energy efficiency. Depending on the underlying technology employed, there are two types of V2X communication technologies: cellular networks and other technologies that support direct device-to-device communication (such as Dedicated Short-Range Communication (DSRC), Port Community System (PCS), Bluetooth^{®}, Wi-Fi^{®}, etc.). Further, the emergency communication apparatus is configured on a computer with the communication function and is connected for bidirectional communication with the on-vehicle emergency report apparatus by a communication line through a radio station and a communication network such as a public telephone network or by satellite communication through a communication satellite. The emergency communication apparatus is adapted to communicate, through the communication network, with communication terminals including a road management office, a police station, a fire department, and a hospital. The emergency communication apparatus can be also connected online with the communication terminals of the persons concerned, associated with the occupant (the driver receiving the service) of the emergency-reporting vehicle.

The term "autonomous vehicle" also referred to as self-driving vehicle, driverless vehicle, robotic vehicle as used herein refers to a vehicle incorporating vehicular automation, that is, a ground vehicle that may sense its environment and move safely with little or no human input. Self-driving vehicles combine a variety of sensors to perceive their surroundings, such as thermographic cameras, Radio Detection and Ranging (radar), Light Detection and Ranging (lidar), Sound Navigation and Ranging (sonar), Global Positioning System (GPS), odometry and inertial measurement unit. Control systems, designed for the purpose, interpret sensor information to identify appropriate navigation paths, as well as obstacles and relevant signage.

The term "rule-based system" as used herein comprises a set of facts of a scenario and a set of rules for how to deal with the set of facts comprising if and then statements, wherein the scenario is predefined in a system.

The term "protocol" as used herein refers to a procedure required to initiate and maintain communication; a formal set of conventions governing the format and relative timing of message exchange between two communications terminals; a set of conventions that govern the interaction of processes, devices, and other components within a system; a set of signaling rules used to convey information or commands between boards connected to the bus; a set of signaling rules used to convey information between agents; a set of semantic and syntactic rules that determine the behavior of entities that interact; a set of rules and formats (semantic and syntactic) that determines the communication behavior of simulation applications; a set of conventions or rules that govern the interactions of processes or applications within a computer system or network; a formal set of conventions governing the format and relative timing of message exchange in a computer system; a set of semantic and syntactic rules that determine the behavior of functional units in achieving meaningful communication; a set of semantic and syntactic rules for exchanging information.

The term "communication protocol" as used herein refers to standardized communication between any two systems. An example communication protocol is of Health Level Seven (HL7). HL7 is a set of international standards used to provide guidance with transferring and sharing data between various healthcare providers. HL7 is a comprehensive framework and related standards for the exchange, integration, sharing, and retrieval of electronic health information.

The term "connection" as used herein refers to a communication link. It refers to a communication channel that connects two or more devices for the purpose of data transmission. It may refer to a physical transmission medium such as a wire, or to a logical connection over a multiplexed medium such as a radio channel in telecommunications and computer networking. The system transfers information through a channel for, for example a digital bit stream, from one or several senders to one or several receivers. A channel has a certain capacity for transmitting information, often measured by its bandwidth in Hertz (Hz) or its data rate in bits per second. For example, a Vehicle-to-Vehicle (V2V) communication may wirelessly exchange information about the speed, location and heading of surrounding vehicles.

The term "alert" or "alert signal" refers to a communication to attract attention. An alert may include visual, tactile, audible alert, and a combination of these alerts to warn drivers or occupants. These alerts allow, drivers or occupants, the ability to act and respond quickly to avoid or navigate through the emergency situation.

The term, "biophysical measurement," as used herein refers to measurement of physical changes that take place over a period of time related to a specific indicator that is measured using an accepted measurement procedure. This provides statistically reliable data that may form the basis for measuring impact and change. Biophysical sensors monitor metabolites, pH, electrolytes, heart rate, arterial oxygenation, sweat rate, and skin temperature, etc., from biophysical signals. It may refer to any signal in living beings that is continually measured and monitored. The term may also be referred to as bio-signal and is often used to refer to bioelectrical signals, but it may refer to both electrical and non-electrical signals. It may refer to time-varying signals, although spatial parameter variations are sometimes subsumed as well.

The term "occupant," as used herein, refers to a passenger in the vehicle and it includes the driver. Passenger and occupant are used interchangeably and refer to a person in the vehicle during a ride.

The term "image sensor" as used herein, refers to a device for recording visual images in the form of photographs, film, or video signals. Image sensor may be a camera and may refer to an infrared camera or a thermographic camera. A thermographic camera is a device that creates an image using infrared (IR) radiation, similar to a normal camera that forms an image using visible light. An infrared camera (also known as a thermal imager) detects and measures the infrared energy of objects. An infrared camera converts infrared data into an electronic image from which surface temperature of the object may be measured.

The term "vehicle seat" as used herein refers to a thing made or used for a person to sit on while traveling in a vehicle. The vehicle seat may be removable. The vehicle seat as referred to herein may be a child car seat that is a removable seat designed to hold a child while riding in the vehicle and the child removable seat usually attaches to a standard seat with hooks or straps.

The term "connector" as used herein refers to a connector that connects geographically separated points. For example, a connector may be a communication connector that transmits information signals. The heart of a communications connector is the transmission medium, which may be a wired or wireless connection. Wired connectors may be optical fibers, coaxial conductors, or twisted wire pairs. Wireless connections may be a Bluetooth connection, Wi-Fi connection or a near field communication connection.

The term "secure connection" as used herein refers to an encrypted connection that has one or more security protocols to ensure the security of data flowing between two or more nodes. When a connection easily listened to by anyone with the knowledge on how to listen in, or an unencrypted connection is even prone to threats by malicious software and rogue and unexpected events is not encrypted or is not a secured connection.

The term "data" as used herein refers to facts and information. The facts and information may be related to an occupant or a vehicle.

The term "signal" as used herein refers to an electrical or electromagnetic current that is used for carrying data from one device or network to another. A signal is a function that conveys information about a phenomenon. Any quantity that can vary over space or time can be used as a signal to share messages between observers. A start signal, a stop signal, an acknowledgement signal are examples of signal. The term start signal refers to a signal that prepares a device to receive data or to perform a function. In asynchronous serial communication, start signals are used at the beginning of a character that prepares the receiving device for the reception of the code elements. The term acknowledgement signal refers to a signal that is passed between communicating processes, computers, or devices to signify acknowledgment, or receipt of message, as part of a communications protocol

The term "predictive diagnostics" as used herein refers to prediction of trends by analyzing the data and predicting trends over the history of the data on the basis of the change in data over a period. For Example, vehicle-specific predictions of components and system conditions in order to optimize the performance of the vehicle, based on status data from the connected vehicle. Sensors continuously monitor the condition of components and systems and transmit the information to the control unit.

The term "cyber security module" as used herein refers to a module comprising application of technologies, processes, and controls to protect systems, networks, programs, devices and data from cyber-attacks and threats. It aims to reduce the risk of cyber-attacks and protect against the unauthorized exploitation of systems, networks, and technologies. It includes, but is not limited to, critical infrastructure security, application security, network security, cloud security, Internet of Things (IoT) security.

The term "encrypt" used herein refers to securing digital data using one or more mathematical techniques, along with a password or "key" used to decrypt the information. It refers to converting information or data into a code, especially to prevent unauthorized access. It may also refer to concealing information or data by converting it into a code. It may also be referred to as cipher, code, encipher, encode. A simple example is representing alphabets with numbers - say, 'A' is '01', 'B' is `02', and so on. For example, a message like "HELLO" will be encrypted as "0805121215," and the system transmits this value over the network to the recipient(s).

The term "decrypt" used herein refers to the process of converting an encrypted message back to its original format. It is generally a reverse process of encryption. It decodes the encrypted information so that only an authorized user may decrypt the data because decryption requires a secret key or password. This term describes a method of unencrypting the data manually or unencrypting the data using the proper codes or keys.

The term "cyber security threat" used herein refers to any possible malicious attack that seeks to unlawfully access data, disrupt digital operations, or damage information. A malicious act includes but is not limited to damage data, steal data, or disrupt digital life in general. Cyber threats include, but are not limited to, malware, spyware, phishing attacks, ransomware, zero-day exploits, trojans, advanced persistent threats, wiper attacks, data manipulation, data destruction, rogue software, malvertising, unpatched software, computer viruses, man-in-the-middle attacks, data breaches, Denial of Service (DoS) attacks, and other attack vectors.

The term "hash value" used herein is as fingerprints for files. A cryptographic algorithm processes the contents of the file, and a unique numerical value, the hash value, is produced that identifies the contents of the file. Modification of the contents in any way, the value of the hash will also change significantly. Example algorithms used to produce hash values: the Message Digest-5 (MD5) algorithm and Secure Hash Algorithm-1 (SHA1).

The term "integrity check" as used herein refers to the checking for accuracy and consistency of system related files, data, etc. It is performed using checking tools that may detect whether any critical system files have been changed, thus enabling the system administrator to look for unauthorized alteration of the system. For example, data integrity corresponds to the quality of data in the databases and to the level by which users examine data quality, integrity, and reliability. Data integrity checks verify that the data in the database is accurate, and functions as expected within a given application.

The term "alarm" as used herein refers to a trigger when a component in a system or the system fails or does not perform as expected. The system may enter an alarm state when a certain event occurs. An alarm indication signal is a visual signal to indicate the alarm state. For example, on detection of a cyber security, the system alerts a system administrator via sound alarm, a message, a glowing LED, a pop-up window, etc. The system reports an alarm indication signal downstream from a detecting device, to prevent adverse situations or cascading effects.

The term "in communication with" as used herein, refers to any coupling, connection, or interaction using electrical signals to exchange information or data, using any system, hardware, software, protocol, or format, regardless of whether the exchange occurs wirelessly or over a wired connection.

As used herein, the term "cryptographic protocol" is also known as security protocol or encryption protocol. It is an abstract or concrete protocol that performs a security-related function and applies cryptographic methods often as sequences of cryptographic primitives. A protocol describes the use of the algorithms. A sufficiently detailed protocol includes details about data structures and representations, at which point it is used to implement multiple, interoperable versions of a program. The system utilizes cryptographic protocols for secure application-level data transport. A cryptographic protocol usually incorporates at least some of these aspects: key agreement or establishment, entity authentication, symmetric encryption, and message authentication material construction, secured application-level data transport, non-repudiation methods, secret sharing methods, and secure multi-party computation. The system utilizes hashing algorithms to verify the integrity of data. The networking switches utilizes cryptographic protocols Secure Socket Layer (SSL) and Transport Layer Security (TLS), the successor to SSL to secure data communications over a network.

As used herein, the term "network" may include the Internet, a local area network, a wide area network, or combinations thereof. The network may include one or more networks or communication systems, such as the Internet, the telephone system, satellite networks, cable television networks, and various other private and public networks. In addition, the connections may include wired connections (such as wires, cables, fiber optic lines, etc.), wireless connections, or combinations thereof. Furthermore, although not shown, other computers, systems, devices, and networks may also be connected to the network. Network refers to any set of devices or subsystems connected by links joining (directly or indirectly) a set of terminal nodes sharing resources located on or provided by network nodes. The computers use common communication protocols over digital interconnections to communicate with each other. For example, subsystems may comprise the cloud. Cloud refers to servers that are accessed over the Internet, and the software and databases that run on those servers.

The problem in today's cars is that there is no way to know if a child, rear facing, is having a medical issue, such as choking, high fever, low vital signs. There needs to be a system that may monitor and provide suggestions when the system detects a health issue.

In cars, there needs to be a system to detect car seats and external seats such as a child car seat and a pet car seat when placed inside the vehicle. The system once detecting the seat may detect the condition of the seat inside the car and the condition of the occupant. The system may generate alerts if the system detects any abnormality in the condition of the seat and the occupant.

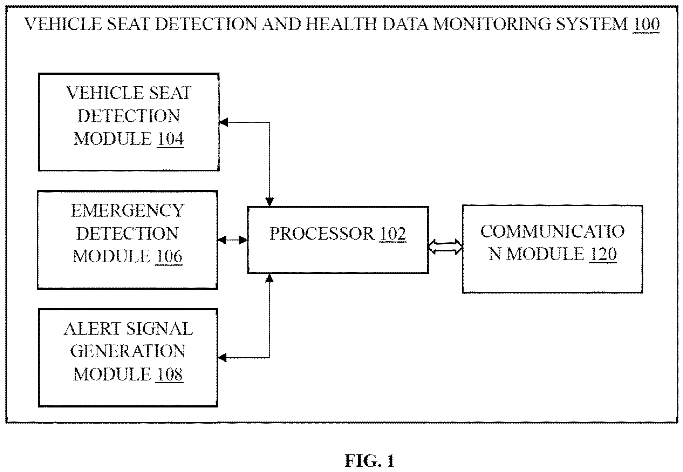

FIG. 1 shows a block diagram of various components of a system for detecting a vehicle seat and monitoring a health emergency of an occupant of the vehicle seat according to an embodiment. The system 100 may comprise a processor 102. In an embodiment, the processor may include a plurality of processors. The system 100 may comprise different modules or subsystems, as shown in FIG. 1, which are communicatively coupled, such as vehicle seat detection module 104, emergency detection module 106, alert signal generation module 108, and communication module 120. In an embodiment, all the modules are configured into a single module or may comprise separate modules.

In an embodiment, the processor is configured to send a start signal to the vehicle seat through the connector, and receive an acknowledgement signal from the vehicle seat via the connector to detect the vehicle seat in the vehicle.

In an embodiment, the processor is configured to receive a signal at the communication module from the vehicle seat, automatically determine if the signal originates from inside the vehicle, if the signal does not originate from inside the vehicle, reject the signal, if the signal originates from inside the vehicle, communicatively connect the communication module to the vehicle seat, receive a message from the vehicle seat, verify that the message originated from inside the vehicle.

In an embodiment, the communication module is enabled for at least one of a vehicle-to-vehicle communication, vehicle-to-infrastructure communication, and vehicle-to-everything communication. The vehicle-to-vehicle communication comprises dedicated short range communication.

In an embodiment, the processor is further configured to perform predictive diagnostics on the first data and the second data using artificial intelligence. The processor is further configured to detect an emergency from the predictive diagnostic performed on the first data and the second data.

An embodiment relates to a method comprising detecting a vehicle seat in the vehicle via the connector, establishing a secure connection with the vehicle seat through the connector, receiving a first data of the vehicle from the sensor, receiving a second data from the camera, processing the first data and the second data, and notifying a condition of the vehicle seat through the communication module.

An embodiment relates to a non-transitory computer storage medium storing a sequence of instructions, which when executed by a processor, causes detecting a vehicle seat in the vehicle via the connector, establishing a secure connection with the vehicle seat through the connector, receiving a first data of the vehicle from the sensor, receiving a second data from the camera, processing the first data and the second data, notifying a condition of the vehicle seat through the communication module.

An embodiment relates to a method comprising detecting a vehicle seat in the vehicle via the connector, establishing a secure connection with the vehicle seat through the connector, receiving a first data of the vehicle from the sensor, receiving a second data from the camera, processing the first data and the second data, and notifying a condition of an occupant through the communication module.

An embodiment relates to a non-transitory computer storage medium storing a sequence of instructions, which when executed by a processor, causes detecting a vehicle seat in the vehicle via the connector, establishing a secure connection with the vehicle seat through the connector, receiving a first data of the vehicle from the sensor, receiving a second data from the camera, processing the first data and the second data and notifying a condition of an occupant through the communication module.

The vehicle seat detection module may detect a vehicle seat inside a vehicle. The vehicle is a car. The vehicle seat may comprise at least one of a normal car seat, a child car seat, and a pet car seat. The vehicle seat comprises at least one of a front facing car seat, rear facing car seat and a booster seat. The vehicle seat is connected to the processor through a connector. The connector is a wired or a wireless connector. Wired connector may comprise an ethernet, Local area network (LAN), Recommended Standard 232 (RS232), Universal serial Bus (USB), Universal Asynchronous Receive Transmit UART or a controller area network (MAY) connection. A wireless connection may comprise a pair of transceivers, near field communication, Bluetooth, or Wi-Fi connection. The vehicle seat may comprise a sensor and a camera. The sensor and the camera may detect a condition of the vehicle seat. In an embodiment, the sensor and the camera may detect a condition of the occupant.

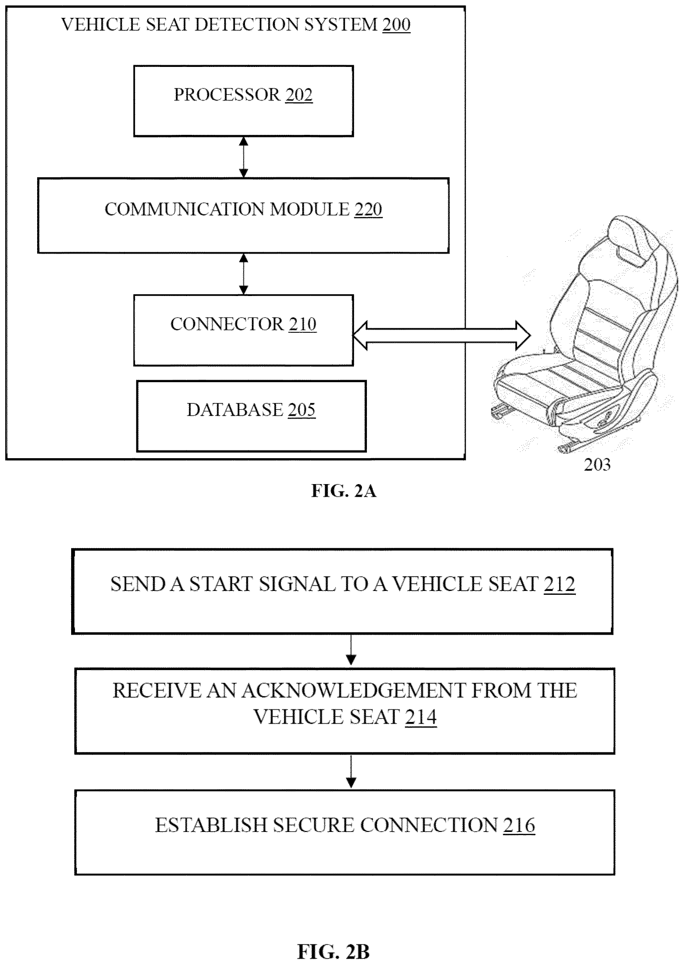

FIG. 2A shows a block diagram for system detection of the vehicle seat 203. When an external vehicle seat 203 is connected into the vehicle such as a child car seat or a pet car seat, the external seat is detected by the vehicle control system. The vehicle seat detection module detects a vehicle seat 203 by exchanging handshaking signals to the vehicle seat through the connector. FIG. 2B shows a flow chart for detection of the vehicle seat. The processor sends a start signal through the connector in order to detect a vehicle seat, at step 212. If there is a vehicle seat attached at the other end of the connector, the processor may receive an acknowledgement signal from the vehicle seat, at step 214. Upon receiving the acknowledgement signal, the processor establishes a secured connection with the vehicle seat, at step 216. The processor may receive a signal at the communication module from the vehicle seat. The processor may further automatically determine if the signal originates from inside the vehicle. If the signal does not originate from inside the vehicle, then the processor is configured to reject the signal. If the signal originates from inside the vehicle, the processor communicatively connects the communication module to the vehicle seat. Then the processor is configured to receive a message from the vehicle seat. Then the processor verifies that the message originated from inside the vehicle. The system studies or analyzes the signals received by the connector 210 (FIG. 2A) to identify the origin location of the signal. As such, the communication transceivers may create a geo-fence (i.e., a virtual perimeter for a realworld geographic area) around the vehicle that allows the communication system to determine whether received signals are currently originating from inside the vehicle.

In an embodiment, the connector 210 includes one or more BLUETOOTH^{™} transceivers to connect to the vehicle seat and the processor. The BLUETOOTH^{™} transceiver is paired with a vehicle seat 203. The BLUETOOTH^{™} transceiver may conduct communications using the BLUETOOTH^{™} protocol with the vehicle seat. Information received from and/or sent to the vehicle seat may originate from the communication module 220.

The connector 210 may also include other communication components that may communicate with different protocols. For example, connector 210 may communicate using an 802.11, 802.11G, or other wireless LAN protocol. The wireless LAN router/antenna may communicate with the vehicle seat 203 and the processor 202. Other types of communication devices or components may include an Ethernet LAN. The Ethernet LAN may include one or more hard-wired ports that connects within the vehicle seat. There is other types of protocols or systems used to communicate with the communication system. The components within the communication system is hardware and/or software and may operate as understood in the art as associated with these communication protocols.

In an embodiment, the system 200 is operable to interface with a single type of communication component and provide those signals to a processor 202. The processor 202 is operable to analyze signal characteristics, relay messages, or do other types of processing received from the vehicle seat. The processor 202 may receive signal data from the vehicle seat through the connector. This data may include timestamps, signal attenuation characteristics, Doppler shift characteristics, and other types of characteristics about the signal. The processor 202 analyzes the signal data may to determine the location of the source of the signal. The system determines whether to provide access to the vehicle to send data to the processor 202 from the location determination.

If the system grants access, the vehicle seat detection module may provide an address to the vehicle seat 203 to provide for inter-device communication or communication from the vehicle to the vehicle seat 203. The processor 202 may also store data about the signal, about the vehicle seat 203 associated with the signal, about the user associated with the signal, or other data in a database 205. The database 205 is any type of data structure or data-storage system, for example, an object-oriented database, flat file database, or other types of databases. The data may include any data received and/or processed by the processor 202 and used to identify the source location of the signals. The processor 202 accesses, stores, or manages the information in the database 205.

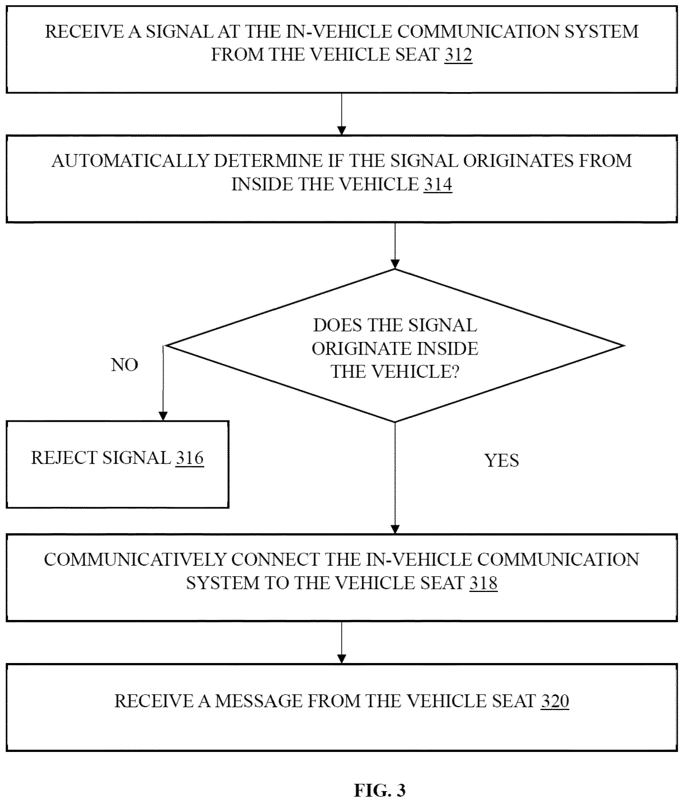

In an embodiment, FIG. 3 shows a method for creating a universal bus for the vehicle system. Generally, the method starts with a start operation and ends with an end operation. The method may include more or fewer steps or may arrange the order of the steps differently. The method is executed as a set of computer-executable instructions executed by a computer system and encoded or stored on a computer readable medium. Hereinafter, the method shall be explained with reference to the systems, components, modules, software, data structures, user interfaces, etc.

The processor may receive a signal from the vehicle seat, in step 312. One of the receivers of the connector receives a signal, which may include a BLUETOOTH^{™} transceiver, an 802.11 transceiver, or some other wired receiver. The system transfers the signal may to the processor through the connector.

In step 314, the processor, through the vehicle seat detection module, may determine if the signal originated from inside the vehicle. the system performs various analyses on the signal or on signal information contained in the signal. If the signal is determined to originate outside the vehicle 315, the method may proceed to step 316 where the processor may reject the receipt of the signal. If the signal is determined to have originated from inside the vehicle, the method may proceed to step 318, where the vehicle system may make a connection to the vehicle seat through the connector and the processor.

The processor, in step 320, may provide an Internet Protocol (IP) address or other type of access such that signals coming from the vehicle seat hereinafter are not rejected. Other types of wireless or wired connections may also be made. If the connection is with a BLUETOOTH^{™} capable vehicle seat, the vehicle system may pair the vehicle seat with a BLUETOOTH^{™} transceiver, in step 320. The vehicle system may make several pairings with the processor as there is two or more BLUETOOTH^{™} transceivers available. Upon making the connection or pairing, the vehicle system may provide access to the communication bus, such that signals to and from the vehicle seat are relayed to the processor of the vehicle system, which is accessed by the vehicle seat sending the signals. In this way, a communication bus is established through wireless or wired connections.

In an embodiment, the processor may also analyze location information. Beyond the signal characteristics, the processor may receive information from sensors 404 to determine a location of the vehicle seat. Further, sensor data is analyzed. Sensor data may include such things as determining if there are people and the number of people within a car. For example, if an optical sensor may view a vehicle seat within its field of vision and/or if an electromagnetic field sensor determines that there is EMF radiation emanating from a location in the vehicle, then the processor may determine that that signal is originating inside a vehicle.

An embodiment relates to a system, wherein a system is configured to be a component of a vehicle. The system comprises a sensor, a camera, a connector, a communication module, and a processor. The processor is configured to detect a vehicle seat in the vehicle via the connector, establish a secure connection with the vehicle seat through the connector, receive a first data of the vehicle from the sensor, receive a second data from the camera, process the first data and the second data, and notify a condition of the vehicle seat through the communication module. The vehicle seat comprises at least one of a normal car seat, a child car seat and a pet car seat. The vehicle seat comprises at least one of a front facing car seat, rear facing car seat and a booster seat. The system comprises a plurality of sensors in the vehicle. The sensor comprises at least one of a seat belt sensor, a position sensor, a pressure sensor, a liquid detection sensor, weight sensor, an infrared sensor, an optical sensor, a comfort detection sensor, a moisture sensor, a temperature sensor, and an audio sensor. The camera comprises an infrared camera, and a thermal camera. The camera is rotatable at an angle to get a clear image. The system comprises a plurality of cameras. The connector comprises at least one of a wired or a wireless connector.

In an embodiment, the first data of the vehicle seat further comprises data related to the vehicle seat position and if the vehicle seat is secured in the right position. The second data comprises an image of the vehicle seat's position and a size of the vehicle seat according to the height and weight of the occupant. The first data comprises at least one of a first position of the vehicle seat, a second position of the occupant on the vehicle seat, a weight of the occupant, a height of the occupant, and a pressure on the seat belt.

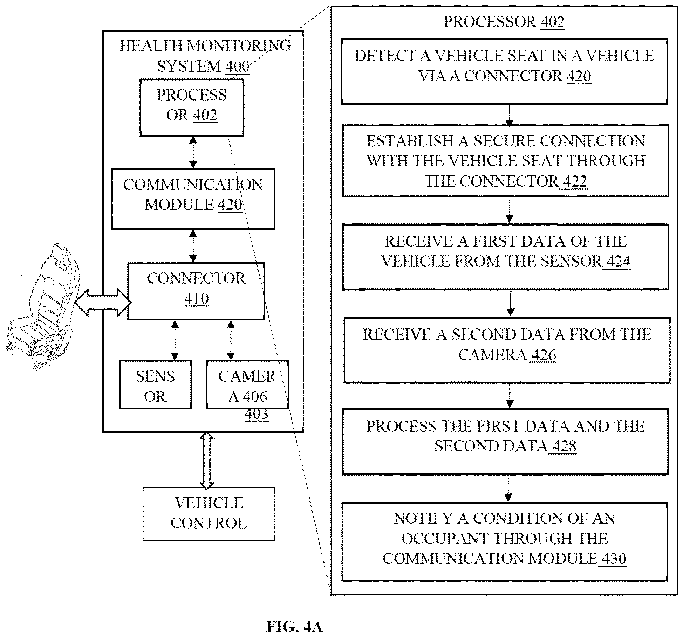

Once the system establishes communication between the vehicle seat 403 and the vehicle control system 401, the vehicle seat may transmit signals related to the occupant's state of health. FIG. 4A shows a system to detect a health risk. The system 400 comprises a sensor 404, a camera 406, and a processor 402. The processor 402 receives data of vehicle seat 403 and health of an occupant from the camera 406 and the sensor 404 through the connector 410.

The processor 402 is configured to perform the folllowing steps: . In an embodiment, at step 420 the system detects a vehicle seat in a vehicle via a connector. The system sends a handshaking signal to the vehicle seat. The system receives an acknowledgement signal if the vehicle seat is connected to the system through the connector. Thus, the system detects the vehicle seat 421.

At step 422, the system establishes a secure connection with the vehicle seat through the connector.

At step 424, the system receives a first data of the vehicle from a sensor. The first data is a physiological condition of the occupant in the vehicle seat.

At step 426, the system receives a second data from a camera. The camera captures the position of the occupant, size of the occupant according to the vehicle seat, seat belt position, conscious state of the occupant.

At step 428, the system processes the first data and the second data, by doing an analysis with a pre-set data of the occupant and detects whether there is a health emergency condition for the occupant or not.

At step 430, the system notifies a user a condition of the occupant through a communication module.

An embodiment relates to a system, wherein a system is configured to be a component of a vehicle. The system comprises a sensor, a camera, a connector, a communication module and a processor. The processor is configured to detect a vehicle seat in the vehicle via the connector, establish a secure connection with the vehicle seat through the connector, receive a first data of the vehicle from the sensor, receive a second data from the camera, process the first data and the second data, and notify a condition of an occupant through the communication module. The vehicle seat comprises at least one of a normal car seat, a child car seat and a pet car seat. The vehicle seat comprises at least one of a front facing car seat, rear facing car seat and a booster seat. The system comprises a plurality of sensors in the vehicle. The sensor comprises at least one of a seat belt sensor, a position sensor, a pressure sensor, a liquid detection sensor, weight sensor, an infrared sensor, an optical sensor, a comfort detection sensor, a moisture sensor, a temperature sensor, and an audio sensor. The camera comprises an infrared camera, and a thermal camera. The sensor for monitoring the health data comprises at least one of a heart rate sensor, a temperature sensor, a blood pressure sensor, a blood presence sensor, and a blood composition sensor. The connector comprises at least one of a wired or a wireless connector.

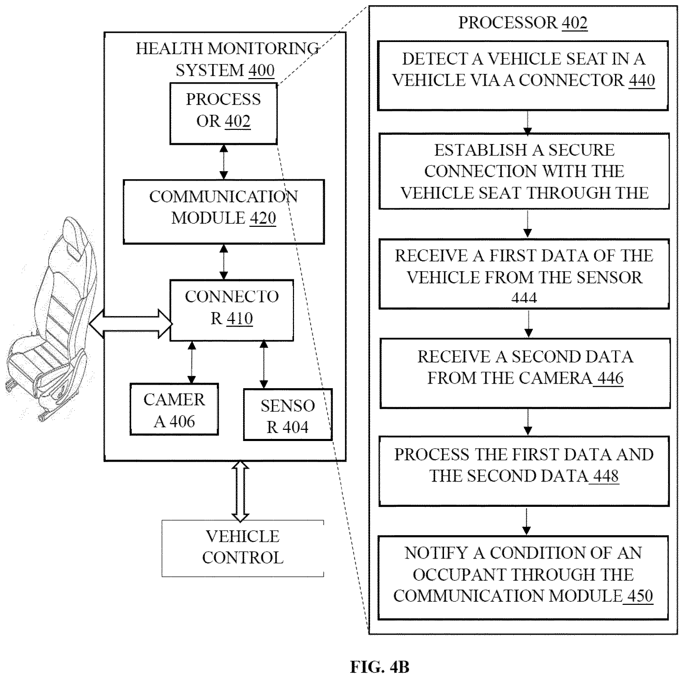

In an embodiment, as shown in FIG. 4B, system 400 comprises a sensor 404, a camera 406, and a processor 402. The processor 402 receives data of vehicle seat 403 from the camera 406 and the sensor 404 through the connector 410.a sensor 404 connected to the vehicle seat recieves a second data of the vehicle seat. The second data comprises an image of the vehicle seat's position, a second position of the occupant on the vehicle seat, and a size of the vehicle seat according to the height and weight of the occupant. The first data comprises at least one of a first position of the vehicle seat, a second position of the occupant on the vehicle seat, a weight of the occupant, a height of the occupant, and a pressure on the seat belt.

The processor 402 is configured to perform the following steps:
At step 442, the system establishes a secure connection with the vehicle seat through the connector.

At step 444, the system receives a first data of the vehicle from a sensor. The first data of the vehicle seat further comprises data related to a vehicle seat position.

At step 446, the system receives a second data from a camera. The camera captures the second data comprises a first image of the vehicle seat position and a second image of a size of the vehicle seat according to a height of an occupant.

At step 448, the system processes the first data and the second data, by doing an analysis of the data of the vehicle seat and detects whether the condition of the vehicle seat is in a good condition or not.

At step 450, the system notifies a user a condition of vehicle seat through a communication module.

The occupant's state of health is determined through image or video processing of the occupant's face and body. For example, the heartbeat of a human is measured with video processing of the green channel for a red, green, blue (RGB) image sensor. In other examples, the occupant monitoring system may detect choking, coughing, sneezing, and sweating through similar video processing methodologies. Using combinations of these information streams, a sophisticated software algorithm may make predictions and suggest an emergency, a severity, and a possible suggestion to address the emergency situation. Using machine learning and video processing, an occupant's weight trends and organ failure (e.g., kidney failure) is determined through changes in the skin color, blotchiness, droopiness, and darkness. The facial expression feature is measured by picking up an image of the face of the occupant by the charge coupled device (CCD) camera, or the like, installed in the vehicle, pointed directly at the face of the occupant, and measuring the facial expression features of the occupant from the image data using the image processing technique. The camera is installed on the interior side of the roof of the vehicle, which may have more than one degree of freedom to monitor the occupant from different angles. In an embodiment, there is a plurality of cameras monitoring the occupant from different angles. The camera detects a conscious state of the occupant, and the position of the occupant.

In an embodiment, it is a system for detecting if a vehicle occupant is experiencing a problematic health situation, which is an emergency situation, and responding to those situations. In the system, sensors in a vehicle monitor, record, and/or detect physiological characteristics of an occupant of the vehicle. Additionally, environmental sensors monitor, record, and/or detect environmental characteristics of the vehicle itself. By detecting the physiological characteristics of the vehicle occupant, receiving physiological characteristic measurements, and using machine learning, the system may determine a baseline for each physiological characteristic corresponding to a specific vehicle occupant (e.g., a driver of the vehicle). The system may then compare newly received physiological characteristic measurements with the baseline for that particular physiological characteristic to determine whether the newly received physiological characteristic measurement is not within the limits of the baseline.

In some embodiments, occupant wellness data may include biophysical data of the occupant. In some embodiments, biophysical data of the occupant is collected or otherwise monitored through one or more biophysical sensors, such as sensors implementable in a vehicle. Sensors is located at various locations in or on the vehicle, in the car seats, in the child car seats, in the belts, on the steering wheel, mirrors, etc. In some embodiments, sensors is in contact with, or worn by, the occupant to monitor or collect biophysical data. For example, a sensor is a heart rate sensor embedded in a seatbelt of the vehicle and configured to provide a heart rate reading of the heart rate of the occupant as biophysical data when the seatbelt is engaged with or worn by the driver. As another example, a sensor is a blood pressure sensor disposed on an armrest of a car seat of the vehicle and configured to provide a blood pressure reading of the blood pressure of the occupant when the occupant places his or her arm on the armrest. Likewise, sensors may include a thermometer, a respiratory rate monitor, a blood glucose monitor, or the like, to provide biophysical data of the occupant, such as a body temperature reading, a respiration reading, a blood glucose reading, or the like. In some embodiments, sensors may include a video camera or an infrared image sensor to capture video(s) and/or image(s) of the occupant and provide imagery data indicative of a body movement, an eye movement, or a facial distortion of the occupant as biophysical data. Using facial detection or other image processing techniques, a processor may analyze the video(s) or image(s) and accordingly determine the emergency situation and its severity. For example, sensors may include a video camera, and processors may analyze a video received from the video camera and find that the occupant may have passed out in the car seat when the system detects no occupant movement. The processor may determine this condition to be very likely an emergency related to the health of the occupant, of which the emergency severity is given a scale of 1-10, where 10 being very severe or life threatening.

In some embodiments, occupant wellness data may also include a medical history of the occupant, and/or a set of emergency-triggering thresholds of the occupant. Medical history may include information on one or more pre-existing medical condition(s) of the occupant, such as hypertension, asthma, or diabetes for example. The system transmits the medical history of the driver from a remote location, such as a cloud server of a medical service provider and received as other data through a communication device thereof. Alternatively, medical history of the occupant is readily stored in memory of the emergency detection system. The processor may analyze the medical history of the occupant while determining emergency severity. For example, the processor may analyze medical history and find that the occupant is a diabetic, and thus may monitor a blood glucose reading of biophysical data received from a blood glucose sensor among sensors. In some embodiments, occupant wellness data of the occupant may include a set of emergency-triggering thresholds associated with the specific occupant, which is provided by a medical doctor or medical service provider. For example, in the case of the diabetic occupant, the set of emergency-triggering thresholds may include a "life-threatening" low-bound blood glucose threshold of 80 mg/dl (milligram per deciliter), and a "non-life-threatening" low-bound blood glucose threshold of 100 mg/dl, as dictated by a medical service provider. In this example, the processor may determine that there is no potential emergency incident should the blood glucose sensor report a reading higher than 100 mg/dl. Moreover, the processor may determine that there is a potential emergency incident of "life-threatening" severity should the blood glucose sensor report a reading lower than 80 mg/dl. Furthermore, the processor may determine that there is a potential emergency incident of "less-than-life-threatening" severity should the blood glucose sensor report a reading between 80 mg/dl and 100 mg/dl.

In still other embodiments, sensors in the car determine where particular passengers or occupant are sitting or located. Sensors may include seat sensors, weight sensors on seats, spring sensors on/in seats, heat sensors on/in seats, motion sensors on/in seats, switches, cameras, heat detectors, or combinations of two or more thereof. The sensor comprises at least one of a seat belt sensor, a position sensor, a pressure sensor, a liquid detection sensor, weight sensor, an infrared sensor, an optical sensor, a comfort detection sensor, a moisture sensor, a temperature sensor, and an audio sensor. The first data of the vehicle seat further comprises data related to the vehicle seat position and if the vehicle seat is secured in the right position. The second data comprises an image of the vehicle seat's position and a size of the vehicle seat according to the height and weight of the occupant. The first data comprises at least one of a first position of the vehicle seat, a second position of the occupant on the vehicle seat, a weight of the occupant, a height of the occupant, and a pressure on the seat belt. In case the size of a vehicle seat is not matching the height of the occupant, the system generates an alert for change of vehicle seat. The system checks the vehicle seat position and if the vehicle seat is not in proper position, an alert for fixing the vehicle seat position is generated. A seat belt pressure sensor checks if the seat belt is fastened properly. In case of a loose seat belt or improper connection of seat belt, the system generates an alert to tighten the seat belt. In an embodiment, the vehicle control system 401 may send signals to tighten the seat belt automatically. In case the occupant is not sitting properly in the vehicle seat, the system generates an alert that the occupant might fall, and the position of the occupant is not proper. In case the vehicle is locked and there is a pet, or a child occupant detected inside the vehicle by the sensors and camera, an alert through an application is generated to the owner of the car to draw their attention. If the position of the vehicle seat is not proper, the system generates an alert will be generated by the alert generation module. If the position of the occupant is not correct and the conscious state of the occupant is detected as not conscious, an alert is generated by the alert generation module. In an embodiment, the condition of the vehicle seat is displayed on a display.

In an embodiment, the condition of the vehicle seat is displayed on a vehicle infotainment system. In an embodiment, the condition of the vehicle seat is displayed on an application. In an embodiment, the condition of the vehicle seat is notified on the vehicle infotainment system.

In an embodiment, the condition of the occupant is displayed on a display. In an embodiment, the condition of the occupant is displayed on a vehicle infotainment system. In an embodiment, the condition of the occupant is displayed on an application.

In some embodiments, the processors may determine emergency severity based on a correlation among occupant wellness data, vehicle motion data, driver wellness data and / or driver distraction or distress data. The vehicle motion data may include various motion parameters of the vehicle, such as a speed of the vehicle, a moving direction of the vehicle, and/or a distance between the vehicle and a nearby object. The system collects and monitors motion data of the vehicle through one or more motion detectors positioned in or on the vehicle. The motion detectors may include one or more of a speedometer, a global positioning device, a video camera, and a proximity sensor.

The alert signal generation module 108 may generate an alert when an emergency is detected. It may further control information displayed on an infotainment system, a rear display, and a front display. A display is designed to display information to different intended users, depending on the positioning of the user's vehicle and the other vehicles. In an embodiment, the display data may include stored display patterns, sequences, colors, text, animation, or other data to be displayed on the front and/or rear display. The display data may also include algorithms for generating content and controlling how it is displayed. The generated content, for example, is personalized based on information received from the system, any third-party system, the vehicle, and the computing devices. In an embodiment, there is indicators outside the vehicle to inform that an emergency is happening, for example a light around the registration plate, a light around the windows of the car. Each color of a light or display may indicate a type of emergency. For example, a red or blue light is configured to indicate an emergency in the health of the vehicle.

In an embodiment, icons on a graphical user interface (GUI) or display of the infotainment system of a computer system are re-arranged based on a priority score of the content of the message. The processor tracks the messages that need to be displayed at a given time and generates a priority score, wherein the priority score is determined based on the action that needs to be taken by the user, the time available before the user input is needed, content of the message to be displayed, criticality of the user's input/action that needs to be taken, the sequence of the message or messages that need to be displayed and executed, and the safety of the overall scenario. For example, in case of a health emergency, the messages in queue for displaying could be an emergency signal, type of emergency, intimation that the system generates an alert to the nearby vehicles.

Vision sensors is coupled with methods such as deep learning methods with region-based Convolutional Neural Networks (R-CNNs) or Fast R-CNNs. In an embodiment, a radar system is used for object-detection which uses radio waves to determine the range, direction, or speed of objects. It is configured to detect motor vehicles. The radar antenna transmits pulses of radio waves which bounce off any object in their path. The object returns a small part of the wave's energy to the receiver antenna which is usually located at the same site as the transmitter. In an embodiment, the sensor that detects the nearby vehicles comprises at least one of an infrared sensor, an ultrasonic sensor, a radar sensor, a passive acoustic detector array, a piezoelectric sensor, a photoelectric sensor, and a camera.

The communication module 420 enables in-vehicle communication, communication with other vehicles, infrastructure communication, grid communication, etc., using Vehicle to network (V2N), Vehicle to infrastructure (V2I), Vehicle to vehicle (V2V), Vehicle to cloud (V2C), Vehicle to pedestrian (V2P), Vehicle to device (V2D), Vehicle to grid (V2G) communication systems. For example, if a child stopped breathing, for some reason, during a trip in a vehicle or car, first, the system detects that a health emergency has happened. Then, the system notifies nearby or surrounding cars or vehicles using the car's communication module. The car or vehicle uses, for example, a message protocol, a message that goes to the other vehicles via a broadcast. In an embodiment, the broadcast message comprises the information of the emergency. In an embodiment, the broadcast message comprises the information of the emergency and the location of the car. In another embodiment, the broadcast message comprises the information of the emergency, the location of the car relative to the car receiving the broadcast message and a pullover target location. In an embodiment, the system creates a graphic from the message received on the receiver's car. In an embodiment, the vehicle communication module sends the broadcast message as soon as the emergency is detected. In another embodiment, the system waits a certain period of time and then broadcasts the message. In another embodiment, the system provides an option to the user if it may broadcast an emergency message. The car experiencing an emergency may actually provide its location. Based on the broadcast message, the receiver of the broadcast message may take action to slow down the vehicle or take any corrective action. In an embodiment, the vehicle experiencing the emergency may automatically override the control of the autonomous vehicle to reduce the speed, change a lane, increase the speed, or a combination thereof. In an embodiment, the system may alert nearby vehicles that there is an emergency in a car and provide a relative location of the car experiencing the emergency to the nearby vehicles. The car experiencing an emergency may send a broadcast message to all the surrounding vehicles to let the vehicles know that the car is in trouble. In an embodiment, the message comprises the type of emergency.

In an embodiment, the health alert system or system provides the images of the person who is undergoing a medical emergency. In an embodiment, the system provides an information after receiving consent from the user of the car.

In an embodiment, the health issue may come from a car that the system has identified and verified that there is a health issue. The system is triggered if it receives information from the car seat or the external device that is inside the car that identifies or detects an emergency inside the car. In an embodiment, the system that enables the health emergency or the emergency alert signal will prevent the abuse of the system when a manual intervention is present.

In an embodiment, the car where the emergency is happening will transmit a message. It is using any or combination of vehicle to vehicle (V2V), vehicle to everything (V2X) or vehicle to infrastructure (V2I) type of communication. In an embodiment, it uses Vehicle-to-vehicle (V2V) communication that enables vehicles to wirelessly exchange information about their speed, location, and heading.

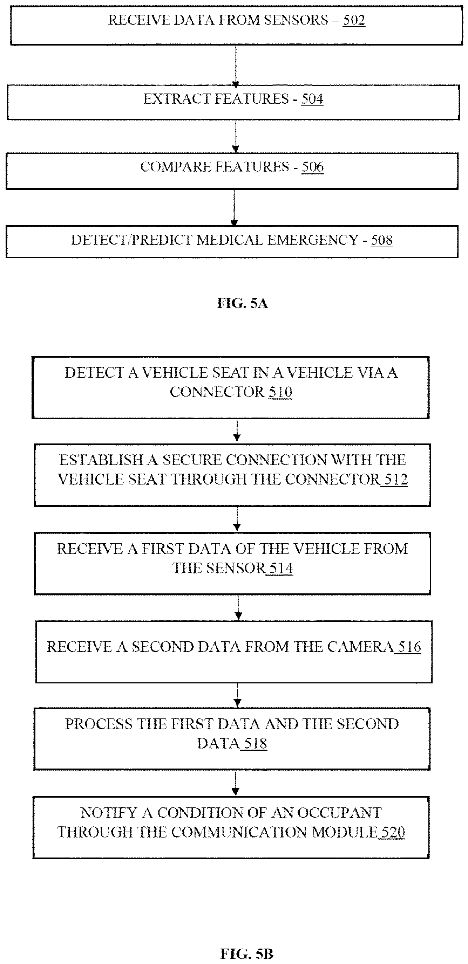

FIG. 5A shows a flowchart for detecting or predicting medical emergency from the sensor data according to an embodiment. According to an embodiment, the emergency detection module 500 comprises the components of an apparatus and the system to detect physiological characteristics of the occupant. The components may each be adapted to measure, track, and/or monitor the occupant by using one or more of the sensors 502 which may comprise camera, microphone, pressure sensors, occupancy sensors, health sensors such as heart rate sensor, blood pressure (BP) sensor, temperature sensor, etc. The data may come from other sources such as a mobile device, a health or fitness tracker, etc. In some embodiments, the system may extract features from the data using machine learning or artificial intelligence algorithm, such features for example may include, but not limited to facial expression, body posture, a voice print, body position and/or motion, vitals - heart rate, respiration rate, body temperature, blood pressure, perspiration, skin color, lip color, comfort level, in vehicle noises, occupant noises, seat occupancy, grip pressure, allergic reaction, etc. as shown at step 504. Occupant noises may include a noise made prior to, during or after the emergency event. A seat occupancy may determine where the occupant is seated, whether the occupant is a child and in a child car seat, etc., and a grip pressure is measured by the force sensors in the car seat or in the belts of the car seat. The processor is enabled to extract a height of the occupant based on the height his head reaches on the seat, the leg resting position, and a posture from the camera. Facial features include eye, mouth, lips, facial muscle, skin, shape of the face etc. Once the features are extracted using image processing algorithm and artificial intelligence, the system uses a pre-trained artificial intelligence/machine learning (AI/ML) model to compare the features at step 506; The system based on the match of features may predict or detect an emergency 508. In an embodiment, the system may predict or detect health emergency related to for example, choking, breathing issue, fever, stroke, heart attack, asthma, seizure, epilepsy, diarrhea, cough, rash, vomiting; loss of consciousness, colic, nosebleed, bleeding, neurological disorder, etc. Example: When a child is experiencing a seizure, some of the symptoms could be body spasms and shaking (a "fit"), loss of awareness or unusual sensations, stiffness or twitching of part of body, such as an arm or hand, smacking lips, rubbing hands, making random noises, moving arms around, picking at clothes or fiddling with objects, chewing, or swallowing, staring blankly into space. By combining the image classification and features recognition algorithm, the health emergency is predicted as a possible seizure. In an embodiment, the system is designed to achieve artificial intelligence (AI) via a model based on predetermined rules, also referred to as rule-based AI system. For example, if the face is drooping (identify from image sensor), arm is numb/weak (identify from image or arm pressure via pressure sensor), speech is slurred (identify via a microphone recording) then provide an emergency alert and indicate that the emergency alert is for possible stroke, with a severity score or scale of 10.

The vehicle control system may also communicate with one or more vehicle sensors 502, which are either associated with the vehicle seat or communicate with the vehicle seat. Vehicle sensors 502 may include one or more sensors for providing information to the vehicle control system that determines or provides information about the environment in which the vehicle is operating. Embodiments of these sensors is as described in conjunction with FIG. 5A. Non-vehicle sensor is any type of sensor that is not currently associated with the vehicle. For example, non-vehicle sensor is sensors in a traffic system operated by a third party that provides data to the vehicle control system. Further, the non-vehicle sensor is other types of sensors which provide information about the distant environment or other information about the vehicle or the environment. These non-vehicle sensors is operated by third parties but provide information to the vehicle control system. Examples of information that is used by the vehicle control system may include weather tracking data, user health tracking data, vehicle maintenance data, or other types of data, which may provide environmental or other data to the vehicle control system.

In an embodiment, a set of sensors or vehicle components associated with the vehicle is as shown in FIG. 5A. The vehicle includes, among many other components common to vehicles, wheels, a power source (such as an engine, motor, or energy storage system (e.g., battery or capacitive energy storage system)), a manual or automatic transmission, a manual or automatic transmission gear controller, a power controller (such as a throttle), a vehicle control system, the display device, a braking system, a steering wheel, a power source activation/deactivation switch (e.g., an ignition), an occupant seating system, a wireless signal receiver to receive wireless signals from signal sources such as roadside beacons and other electronic roadside devices, and a satellite positioning system receiver (e.g., a Global Positioning System ("GPS") (US), GLONASS (Russia), Galileo positioning system (EU), Compass navigation system (China), and Regional Navigational Satellite System (India) receiver).

The vehicle includes a number of sensors in wireless or wired communication with the vehicle control system and/or display device to collect sensed information regarding the vehicle state, configuration, and/or operation. Exemplary sensors include wheel state sensor to sense one or more of vehicle speed, acceleration, deceleration, wheel rotation, wheel speed (e.g., wheel revolutions-per-minute), wheel slip, and the like, a power source energy output sensor to sense a power output of the power source by measuring one or more of current engine speed (e.g., revolutions-per-minute), energy input and/or output (e.g., voltage, current, fuel consumption, and torque, turbine speed sensor, input speed sensor, crankshaft position sensor, manifold absolute pressure sensor, mass flow sensor), and the like, a switch state sensor to determine a current activation or deactivation state of the power source, activation/deactivation switch, a transmission setting sensor to determine a current setting of the transmission (e.g., gear selection or setting), a gear controller sensor to determine a current setting of the gear controller, a power controller sensor to determine a current setting of the power controller (such as a throttle), a brake sensor to determine a current state (braking or non-braking) of the braking system, a seating system sensor to determine a seat setting and current weight of seated occupant in a selected seat of the seating system, exterior and interior sound receivers (e.g., a microphone and other type of acoustic-to-electric transducer or sensor) to receive and convert sound waves into an equivalent analog or digital signal. Examples of other sensors (not shown) that is employed include safety system state sensors to determine a current state of a vehicular safety system (e.g., air bag setting (deployed or undeployed) and/or seat belt setting (engaged or not engaged)), light setting sensor (e.g., current headlight, emergency light, brake light, parking light, fog light, interior or passenger compartment light, and/or tail light state (on or off)), brake control (e.g., pedal) setting sensor, accelerator pedal setting or angle sensor, clutch pedal setting sensor, emergency brake pedal setting sensor, door setting (e.g., open, closed, locked or unlocked) sensor, engine temperature sensor, passenger compartment or cabin temperature sensor, window setting (open or closed) sensor, one or more cameras or other imaging sensors (which commonly convert an optical image into an electronic signal but may include other devices for detection of objects such as an electromagnetic radiation emitter/receiver (that emits electromagnetic radiation and receives electromagnetic waves reflected by the object) to sense objects, such as other vehicles and pedestrians and optionally determine the distance, trajectory and speed of such objects, in the vicinity or path of the vehicle), odometer reading sensor, trip mileage reading sensor, wind speed sensor, radar transmitter/receiver output, brake wear sensor, steering/torque sensor, oxygen sensor, ambient lighting sensor, vision system sensor, ranging sensor, parking sensor, heating, venting, and air conditioning (HVAC) sensor, water sensor, air-fuel ratio meter, blind spot monitor, hall effect sensor, microphone, radio frequency (RF) sensor, infrared (IR) sensor, wireless network sensor (e.g., Wi-Fi and/or BLUETOOTH^{™} sensor), cellular data sensor, and other sensors known to those of skill in the vehicle art.

In the depicted vehicle embodiment, the various sensors are in communication with the display device and vehicle control system via signal carrier network. As noted, the signal carrier network is a network of signal conductors, a wireless network (e.g., a radio frequency, microwave, or infrared communication system using a communications protocol, such as Wi-Fi), or a combination thereof.

Other sensors included and positioned in the interior space of the vehicle. Generally, these interior sensors obtain data about the health of the driver and/or passenger(s), data about the safety of the driver and/or passenger(s), and/or data about the comfort of the driver and/or passenger(s). The health data sensors may include sensors in the steering wheel that may measure various health telemetry for the person (e.g., heart rate, temperature, blood pressure, blood presence, blood composition, etc.). Sensors in the seats may also provide for health telemetry (e.g., presence of liquid, weight, weight shifts, etc.). Infrared sensors could detect a person's temperature; optical sensors may determine a person's position and whether the person has become unconscious. Other health sensors are possible and included herein.

Safety sensors may measure whether the person is acting safely. Optical sensors may determine a person's position and focus. If the person stops looking at the road ahead, the optical sensor may detect the lack of focus. Sensors in the seats may detect if a person is leaning forward or injured in a collision. Other sensors may detect that the driver has at least one hand on a steering wheel. Other safety sensors are possible and contemplated as if included herein.

Comfort sensors may collect information about a person's comfort. Temperature sensors may detect a temperature of the interior cabin. Moisture sensors may determine relative humidity. Audio sensors may detect loud sounds or other distractions. Audio sensors may also receive input from a person through voice data. Other comfort sensors are possible and contemplated as if included herein.

FIG. 5B shows a method for receiving alerts and health data of passengers according to an embodiment. In an embodiment, at step 510 the system detects a vehicle seat in a vehicle via a connector. The system sends a handshaking signal to the vehicle seat. The system receives an acknowledgement signal if the vehicle seat is connected to the system through the connector. Thus, the system detects the vehicle seat.

At step 512, the system establishes a secure connection with the vehicle seat through the connector.

At step 514, the system receives a first data of the vehicle from a sensor. The first data is a physiological condition of the occupant in the vehicle seat.

At step 516, the system receives a second data from a camera. The camera captures the position of the occupant, size of the occupant according to the vehicle seat, seat belt position, conscious state of the occupant.

At step 518, the system processes the first data and the second data, by doing an analysis with a pre-set data of the occupant and detects whether there is a health emergency condition for the occupant or not.

At step 520, the system notifies a user a condition of the occupant through a communication module.

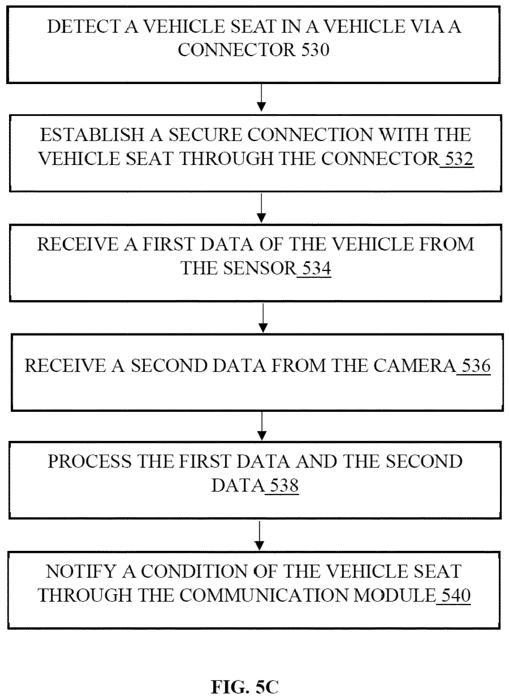

FIG. 5C shows a method for receiving alerts and condition of vehicle seat according to an embodiment. In an embodiment, at step 530 the system detects a vehicle seat in a vehicle via a connector. The system sends a handshaking signal to the vehicle seat. The system receives an acknowledgement signal if the vehicle seat is connected to the system through the connector. Thus, the system detects the vehicle seat.

At step 532, the system establishes a secure connection with the vehicle seat through the connector.

At step 534, the system receives a first data of the vehicle from a sensor. The first data of the vehicle seat further comprises data related to a vehicle seat position.

At step 536, the system receives a second data from a camera. The camera captures the second data comprises a first image of the vehicle seat position and a second image of a size of the vehicle seat according to a height of an occupant.

At step 538, the system processes the first data and the second data, by doing an analysis of the data of the vehicle seat and detects whether the condition of the vehicle seat is in a good condition or not.

At step 540, the system notifies a user a condition of vehicle seat through a communication module.

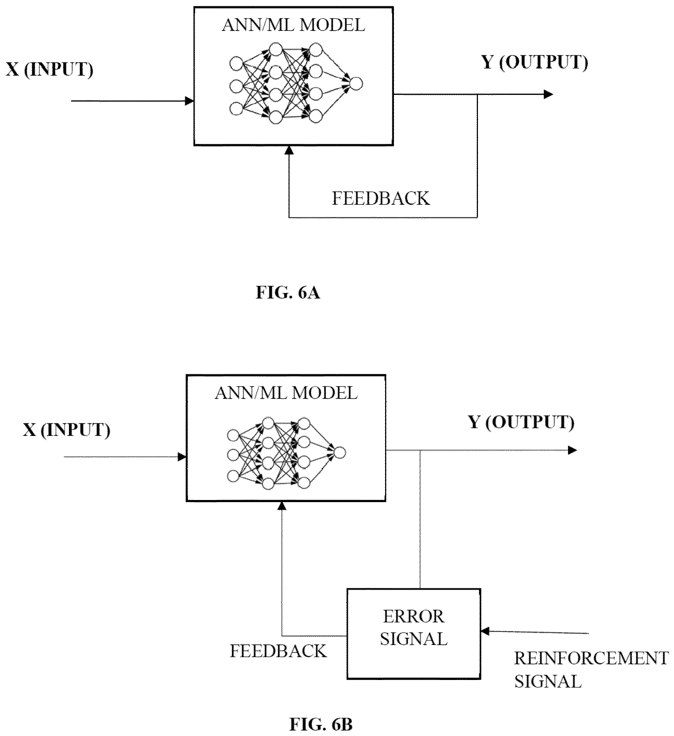

FIG. 6A shows a structure of the neural network / machine learning model with a feedback loop. Artificial neural networks (ANNs) model comprises an input layer, one or more hidden layers, and an output layer. Each node, or artificial neuron, connects to another and has an associated weight and threshold. If the output of any individual node is above the specified threshold value, activates that node, sending data to the next layer of the network. Otherwise, no data is passed to the next layer of the network. A machine learning model or an ANN model is trained on a set of data to take a request in the form of input data, make a prediction on that input data, and then provide a response. The model may learn from the data. Learning is supervised learning and/or unsupervised learning and is based on different scenarios and with different datasets. Supervised learning comprises logic using at least one of a decision tree, logistic regression, support vector machines. Unsupervised learning comprises logic using at least one of a k-means clustering, a hierarchical clustering, a hidden Markov model, and an apriori algorithm. The output layer may predict or detect a health issue and the severity of the health issue based on the input data.

In an embodiment, ANN's is a Deep-Neural Network (DNN), which is a multilayer tandem neural network comprising Artificial Neural Networks (ANN), Convolution Neural Networks (CNN) and Recurrent Neural Networks (RNN) that may recognize features from inputs, do an expert review, and perform actions that require predictions, creative thinking, and analytics. In an embodiment, ANNs is Recurrent Neural Network (RNN), which is a type of Artificial Neural Networks (ANN), which uses sequential data or time series data. Deep learning algorithms are commonly used for ordinal or temporal problems, such as language translation, Natural Language Processing (NLP), speech recognition, and image recognition, etc. Like feedforward and convolutional neural networks (CNNs), recurrent neural networks utilize training data to learn. They are distinguished by their "memory" as they take information from prior input via a feedback loop to influence the current input and output. An output from the output layer in a neural network model is fed back to the model through the feedback. The variations of weights in the hidden layer(s) will be adjusted to fit the expected outputs better while training the model. This will allow the model to provide results with far fewer mistakes.

The neural network is featured with the feedback loop to adjust the system output dynamically as it learns from the new data. In machine learning, backpropagation and feedback loops are used to train an AI model and continuously improve it upon usage. As the incoming data that the model receives increases, there are more opportunities for the model to learn from the data. The feedback loops, or backpropagation algorithms, identify inconsistencies and feed the corrected information back into the model as an input.

Even though the AI/ML model is trained well, with large sets of labeled data and concepts, after a while, the models' performance may decline while adding new, unlabeled input due to many reasons which include, but not limited to, concept drift, recall precision degradation due to drifting away from true positives, and data drift over time. A feedback loop to the model keeps the AI results accurate and ensures that the model maintains its performance and improvement, even when new unlabeled data is assimilated. A feedback loop refers to the process by which an AI model's predicted output is reused to train new versions of the model.

Initially, when the AI/ML model is trained, a few labeled samples comprising both positive and negative examples of the concepts (for e.g., health issues) are used that are meant for the model to learn. Afterward, the model is tested using unlabeled data. By using, for example, deep learning and neural networks, the model may then make predictions on whether the desired concept/s (for e.g., detection of health issue) are in unlabeled images. Each image is given a probability score where higher scores represent a higher level of confidence in the models' predictions. Where a model gives an image a high probability score, it is auto labeled with the predicted concept. However, in the cases where the model returns a low probability score, this input is sent to a controller (is a human moderator) which verifies and, as necessary, corrects the result. The human moderator is used only in exception cases. The feedback loop feeds labeled data, auto-labeled or controller-verified, back to the model dynamically and is used as training data so that the system may improve its predictions in real-time and dynamically.

FIG. 6B shows a structure of the neural network / machine learning model with reinforcement learning. The network receives feedback from authorized networked environments. Though the system is similar to supervised learning, the feedback obtained in this case is evaluative not instructive, which means there is no teacher as in supervised learning. After receiving the feedback, the network performs adjustments of the weights to get better predictions in the future. Machine learning techniques, like deep learning, allow models to take labeled training data and learn to recognize those concepts in subsequent data and images. The new data is fed into the model for testing, hence by feeding the model with data it has already predicted over, the training gets reinforced. If the machine learning model has a feedback loop, the learning is further reinforced with a reward for each true positive of the output of the system. Feedback loops ensure that AI results do not stagnate. By incorporating a feedback loop, the model output keeps improving dynamically and over usage/time.

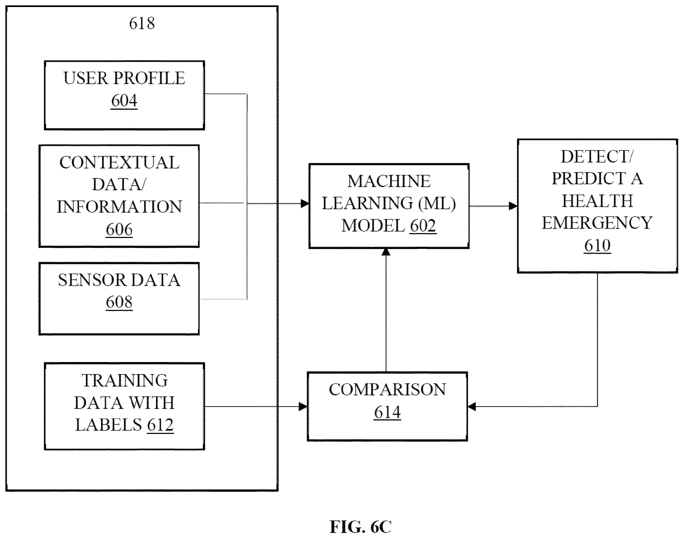

FIG. 6C shows an example block diagram for detecting a health emergency using a machine learning model. The machine learning model 602 may take as input any data associated with the occupant and learn to identify features within the data that are predictive of personalization preferences. The training data sample may include, for example, the occupant's profile data 604, such as the age, gender, weight, height, ethnicity, demographic location, residence location, work location, past health conditions, data from health records and any other suitable data relating to the occupant. In an embodiment, the machine learning model relates to systems and methods that trigger a vehicle emergency event or the like, capture the health status of a previously identified or unidentified vehicle occupant in real time using an on-board camera and/or other sensors. The system transmits the vehicle occupant identification and health status information to the cloud, where the vehicle occupant identification is coupled with available public health profile data in the case of a previously identified vehicle occupant. Subsequently, the system transmits the health status information and public health profile data to an emergency responder or the like, thereby aiding in rendering medical assistance to an injured or sick vehicle occupant. The systems and methods of the present disclosure may also provide data analytics information that is used later to improve vehicle safety. When a vehicle occupant enters a vehicle, he, or she the vehicle system identifies the occupant using an interior camera and a facial recognition algorithm, a near-field mobile device identification methodology, a self-identification user interface, or the like. The one or more pre-populated potential vehicle occupant databases resident in the cloud subsystem supplements the identification process. In any event, the vehicle subsystem provides a real time vehicle occupant list or map (also generated using one or more on-board vehicle occupant position sensors, or the like) that is transmitted to and maintained by the cloud subsystem. This real time vehicle occupant list or map is associated with a given vehicle identifier, for example. Thus, at all times, the cloud subsystem monitors who is in a particular vehicle and, preferably, where they are seated in the vehicle. In an embodiment, there is a vehicle occupant matching algorithm which is in the vehicle system or is remote from the vehicle system in a cloud environment. The vehicle system transmits a unique vehicle occupant ID to the vehicle occupant matching algorithm for each sensed/imaged vehicle occupant. The system transmits the vehicle occupant updates to the cloud system for maintaining the vehicle occupant ride lists, associating the appropriate public health profiles, and providing them to emergency responders or other appropriate recipients, accordingly.

In an embodiment, the training data sample may also include current contextual information 606 relating to the ride. This may include, for example, location of the vehicle, current weather condition, temperature, time of day, traffic condition in the region, seating position or location within the vehicle, posture of the occupant, etc. The system may also garner contextual information 606 from a device in the vehicle or a device of the occupant. For example, through an application installed on the device, for example, google maps and location services, the system may know the ride details. For example, the system obtains the occupants state of mind from an image of the camera where eye pattern is monitored, a sensor where heart rate is monitored. The system may correlate the eye movements with the health data. In an embodiment, the application may access other types of application usage data from other applications (including the operating system) installed on the occupant's device and use them as contextual information 606. This may include other applications that are being used before and/or during the ride (e.g., whether it was a gaming application, a video or productivity application). In an embodiment, a microphone installed in the vehicle or on an external device in the vehicle may record the noises and conversation of the occupants. The application may also query the other applications for data relating to, for example, age, gender, circle of friends, family, user destination, emergency contacts, the friends' interests, vehicle data, trip information, etc. Vehicle data, such as speed and trip data such as origin and destination, and in between stops may further indicate whether the occupant is going home from work, going to work from home, picking children up, running an errand, etc., which may also serve as contextual information 606.

Real-time sensor data 608 may include, for example, video, image, audio, infrared, temperature, 3D modeling, and any other suitable types of data that capture the occupant's current state. In an embodiment, the real-time sensor data is processed using one or more machine learning models 602 trained and based on similar types of data to predict real-time features of the occupant. The real-time features may include, for example, the occupant's current mood (e.g., happy, angry, sad, etc.), stress level, comfort level with respect to vehicle amenities (e.g., temperature, audio, entertainment, etc.), health condition, and/or any other features that may characterize or represent the occupant's current state. Current information about the occupant is used for the detection of health issues or emergencies. For example, currently detected sensor data and/or previously known information about the occupant is used to predict the occupant's health condition.

Any of the aforementioned types of data (e.g., occupant's profile data 604, contextual information 606, sensor data 608, or any other data) may correlate with the occupant's general health condition and disposition, and such correlation is automatically learned by the machine learning model 602. In an embodiment, during training, the machine learning model 602 may process the training data sample (e.g., occupant's profile data 604 and/or contextual information 606) and, based on the current parameters of the machine learning model 602, detect or predict a health emergency 610. The detection or prediction of an emergency 610 may depend on the training data with labels 612 associated with the training sample 618. Predicting a health issue refers to predicting a future event based on past and present data and most commonly by analysis of trends or data patterns. Prediction or predictive analysis employs probability based on the data analyses and processing. Detection of a health emergency refers to an onset of an emergency and the system detecting the same. Based on the turn of events, predicted events may not turn into emergencies. For example, the system based on a color of the skin appearing bluish may predict a breathing issue or a choking, but it may not turn into an emergency if the occupant starts recovering. However, if the skin color is not improving, then the system detects that the occupant is experiencing a breathing issue/ choking issue. For example, if the training label 612 indicates a particular type of health condition, e.g., diabetes, a seizure, a stroke, the machine learning model 602 would learn to detect the issue based on input data associated with a given occupant's profile data 604, contextual information 606 and/or sensor data 608. In an embodiment, during training, the detected health emergency 610, and the system compares the training data with labels 612 in comparison block 614. For example, the comparison block 614 is based on a loss function that measures a difference between the detected health emergency 610 and the training data with labels 612. Based on the comparison block 614 or the corresponding output of the loss function, a training algorithm may update the parameters of the machine learning model 602, with the objective of minimizing the differences or loss between subsequent predictions or detections of the health emergencies 610 and the corresponding labels 612. By iteratively training in this manner, the machine learning model 602 may "learn" from the different training data samples and become better at detecting various health emergencies at that are similar to the ones represented by the training labels 612. In an embodiment, the system trains the machine learning model 602 using data which is specific to the occupant for which the model is used for detecting a health emergency. In an embodiment, the system trains machine learning model 602 using data which is general to the health condition and is used for an occupant for detecting a health emergency.

In an embodiment, sensor data 608 from the vehicle that is associated with a time segment (e.g., 5, 10, 30, or 60 seconds) is labeled or associated with one or more predetermined event types to represent what transpired or occurred in the vehicle during that time segment. For example, a particular training data sample 618 that is capturing a health emergency occurring in a vehicle is labeled 612 and associated with a "health emergency" event type. For example, a particular training data sample 618 may pertain to a particular incident that occurred in the past. The training data sample 618 is labeled 612 or associated with the "health emergency" event type, and the sensor data 608 may include audiovisual data of the event as it was occurring, along with profile data 604 of the occupant. In an embodiment, the training data sample 618 may further include contextual information 606, such as the time of day of the ride, the weather, app-usage patterns associated with the ride request, etc.

Using the training data, a machine learning model 602 is trained so that it recognizes features of input data that signify or correlate to certain event types. For example, a trained machine learning model 602 may recognize data features that signify the likelihood of an emergency situation, as an actionable event. In an embodiment, the features may have meaningful interpretations, such as excessive physical movements, no physical movement, clutching at the throat, inability to speak, breathe or swallow, coughing, wheezing or other unusual breathing sounds, gagging, a change in color (e.g., blue lips or red face), audible or gestured request for help, etc. In an embodiment, the symptoms of the disease or health emergency are used as training data or input data for training. For example, symptoms of rapid heart rate, bluish skin symptoms of hypoxemia will be used for mapping the symptoms to the disease hypoxemia. The features may also be unintelligible to humans and may simply represent data patterns that tend to be present when certain event types occur. Through training, the machine learning model 602 may learn to identify predictive and non-predictive features and apply the appropriate weights to the features to optimize the machine learning model's predictive accuracy. In embodiments where supervised learning is used and each training data sample 618 has a health emergency label 612, the training algorithm may iteratively process each training data sample 618 (including user profile data 604, contextual information 606, and/or sensor data 608), and generate a prediction of health emergency 610 based on the model's 602 current parameters. Based on the comparison block 614 results, the training algorithm may adjust the model's 602 parameters/configurations (e.g., weights) accordingly to minimize the differences between the generated predictions 610 and the corresponding labels 612. Any suitable machine learning model and training algorithm is used, including, e.g., neural networks, decision trees, clustering algorithms, and any other suitable machine learning techniques. Once trained, the machine learning model 602 may take input data associated with an occupant and output one or more predictions that indicate a likelihood that a health emergency event has occurred. In an embodiment, the occupant and the vehicle associated with the input data is different from any of the occupants and vehicles associated with the training data samples.

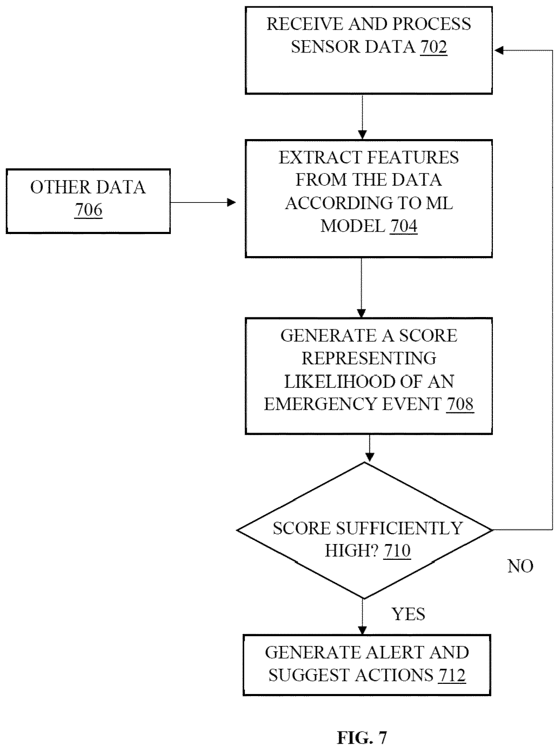

FIG. 7 shows an example flow chart for detecting a health emergency using a machine learning model. The system may receive data associated with sensor output(s) from one or more sensors in the vehicle as shown at step 702. Any type of sensor is used to gather data pertaining to the passenger compartment of the vehicle. A sensor output is, for example, images, videos, audios, LiDAR measures, infrared measures, temperature measures, GPS data, or any other information measured or detected by sensors. In an embodiment, a sensor output is the result of one or more sensors capturing environmental information associated with the passenger compartment of the vehicle, which may include the occupant, driver, and any other passengers/occupants of the vehicle. For example, sensor output is associated with an occupant in the vehicle including the driver. In an embodiment, data associated with sensor output may include the raw sensor output (e.g., the images, videos, audios, etc.) and/or data derived from processing the sensor output. For example, the vehicle's computer system, a vehicle device, or a cloud/network system may process the sensor data and generate derivative data. For example, derivative data from audio may include data representing pitch, tone, speech rate, and/or semantic information. As another example, derivative data from image, video or LiDAR data may include data representing identified objects, motion, movement speed, gestures, facial expressions, posture, eye position, etc. The system may receive any data associated with the sensor output from sensors, including raw sensory output and/or any derivative data. In an embodiment, the system may process the received data and identify any actionable event of interest using a machine learning model, trained using a set of training data.

As shown at step 704, the system may extract features from the received data according to a machine learning model. The machine learning model is able to extract features automatically, based on what it learned during the training process. In an embodiment, appropriate weights that were learned during the training process is applied to the features. At step 708, the machine learning model, based on the features of the received data, may generate a score representing a likelihood or confidence that the received data is associated with a particular event type, e.g., health emergency due to choking, health emergency due to hypoxemia, or simply health emergency, etc. As shown at step 710, the system may determine whether the score is sufficiently high relative to a threshold or criteria to warrant certain action. If the score is not sufficiently high, thus indicating that the detected event may not have actually occurred (in other words, a false-positive), the system may return to step 702 and continue to monitor subsequent incoming data. On the other hand, if the score is sufficiently high, then at step 712 the system may generate an appropriate alert and/or determine an appropriate action/response. In an embodiment, the system may send alerts to appropriate recipients based on the detected event types.

For instance, the system generates an alert, and a message is sent to nearby vehicles. In an embodiment, the system sends an alert automatically of a health emergency sent to a nearby hospital, ambulatory service, and/or firefighters. The system sends an alert additionally or alternatively to one of the devices within the vehicle in which the event is occurring. For example, the alert may warn the passengers that events in the vehicle are being recorded, inform the passengers that an appropriate third-party has been contacted (e.g., police, ambulance, etc.), or provide guidance on what to do (e.g., remain calm in the vehicle, continue to nearby emergency care with step-by-step instructions, etc.). In situations where the driver of the vehicle is found to be in a panic or distressed due to the health emergency of an occupant in the car or driver himself/herself having a health emergency, an autonomous vehicle mode is initiated, and the system may also instruct an autonomous vehicle to perform certain actions. For example, the system may determine an alternative destination different from the originally intended destination and instruct the autonomous vehicle to drive to that alternative destination. For instance, the system instructs the vehicle to drive to the nearest hospital in the event of a health emergency.

In an embodiment, the system may repeat one or more steps of the method of FIG. 7, where appropriate. In an embodiment, the steps 702 to 712 are performed by the system, any combination of those steps is performed by any other computing system, for example a remote network or a cloud network. In an embodiment, where machine learning models are used for making such determination, the system may transmit a trained machine learning model to the computing system in the vehicle to allow event-detection to be made locally. This is desirable since sensor data is overly large to transmit to the remote system in a timely fashion. If the machine learning model takes as input other data 706 (e.g., the occupant's medical data, medical preconditions, his social habits such as drinking, partying, sleeping habits, etc.), such information is made available to the computing device executing the machine learning model (e.g., the computing device may obtain the data from the occupant's device). In an embodiment, the local computing device may send the event-detection determination to the system and let it perform the appropriate actions (e.g., generate alerts, etc., as described with reference to step 712).

In an embodiment, the system is provided, wherein the facial expression recognition module utilizes a Convolutional Neural Networks (CNN) pre-training process; and/or wherein the machine learning (ML) algorithm contains a bounding box procedure around the subject's face; and/or wherein the bounding box procedure utilizes a Viola-Jones detection algorithm; and/or wherein the facial expression recognition module utilizes Facial Expression Recognition (FER) algorithms; and/or wherein the current behavioral classification is of emotional states of at least one of anger, happiness, and calm; and/or wherein the current behavioral classification is indicative of a health emergency; and/or wherein the current behavioral classification requires an action; and/or wherein the alerting is at least one of a light, a sound, an electronic message; and/or wherein the camera is a video camera. In yet another aspect of the disclosure, the above method is provided, wherein the step of learning utilizes a Convolutional Neural Networks (CNN) pre-training process; and/or wherein the detecting of a face of an occupant in the images is via a bounding box procedure; and/or further comprising using a Viola-Jones detection algorithm; and/or wherein the current behavioral classification is at least one of anger, happiness, and of a health emergency; and/or wherein the occupant experiences a health emergency; and/or wherein the step of alerting is via at least one of a light, a sound, an electronic message displayed via a display in the vehicle. In an embodiment, one or more currently obtained facial expressions from the camera are compared to thresholds for pre-trained emergency classifications and determine when a current classification is an emergency. When the system detects the current emergency classification, requiring an action, a driver is alerted, the system signals directly to provide real-time assistance. For instance, when the user's heart rate surpasses a predetermined threshold, the system identifies and classifies the instance as an emergency. In an embodiment, the thresholds are varied. In an embodiment, a Long-Short Term Memory-based Recurrent Neural Network module is used, with current image or video sequences, to predict an emergency when a user's facial expression changes (based on a threshold). The use of a recurrent neural network architecture employs its ability to use past, temporal information for inference on current inputs. Long short-term memories (LSTMs) offer a computationally efficient way to train these networks. For example, video sequences of individuals in emergency events are used to train the LSTMs. By virtue of this training mechanism, the model may predict, given real time video input, when an emergency is imminent.

In an embodiment, multiple sensors are utilized to gather information and thereafter develop "intelligence" on the occupant. In an embodiment, the system uses only image or video data to assess the person(s)' state or condition which is tailored to be non-contact monitoring. The system monitors the state of the occupants and, primarily via extracted facial expressions and changes of facial features with Artificial Intelligence (AI) assistance, is able to provide changes or predictions to the state of health in real time or in advance. The system is configured for continuous monitoring. The features are processed with neural network algorithms with temporal modeling to capture primary and micro facial expressions to help detect and predict significant behavioral changes of the occupant's emotional and health state. In an embodiment, the system may recommend a treatment option through machine learning and prediction methodologies.

In an embodiment, the system further comprises a cyber security module wherein the cyber security module comprises an information security management module providing isolation between the communication module and servers.

In an embodiment, the information security management module is operable to, receive data from the communication module, exchange a security key at a start of the communication between the communication module and the server, receive the security key from the server, authenticate an identity of the server by verifying the security key, analyze the security key for a potential cyber security threat, negotiate an encryption key between the communication module and the server, encrypt the data; and transmit the encrypted data to the server when no cyber security threat is detected.

In an embodiment, the information security management module is operable to exchange a security key at a start of the communication between the communication module and the server, receive the security key from the server, authenticate an identity of the server by verifying the security key, analyze the security key for a potential cyber security threat, negotiate an encryption key between the system and the server, receive encrypted data from the server, decrypt the encrypted data, perform an integrity check of the decrypted data and transmit the decrypted data to the communication module when no cyber security threat is detected.

In an embodiment, the system may comprise a cyber security module.

In one aspect, a secure communication management (SCM) computer device for providing secure data connections is provided. The SCM computer device includes a processor in communication with memory. The processor is programmed to receive, from a first device, a first data message. The first data message is in a standardized data format. The processor is also programmed to analyze the first data message for potential cyber security threats. If the determination is that the first data message does not contain a cyber security threat, the processor is further programmed to convert the first data message into a first data format associated with the vehicle environment and transmit the converted first data message to the vehicle system using a first communication protocol associated with the vehicle system.

According to an embodiment, secure authentication for data transmissions comprises, provisioning a hardware-based security engine (HSE) located in communications system, said HSE having been manufactured in a secure environment and certified in said secure environment as part of an approved network; performing asynchronous authentication, validation and encryption of data using said HSE, storing user permissions data and connection status data in an access control list used to define allowable data communications paths of said approved network, enabling communications of the communications system with other computing system subjects to said access control list, performing asynchronous validation and encryption of data using security engine including identifying a user device (UD) that incorporates credentials embodied in hardware using a hardware-based module provisioned with one or more security aspects for securing the system, wherein security aspects comprising said hardware-based module communicating with a user of said user device and said HSE.

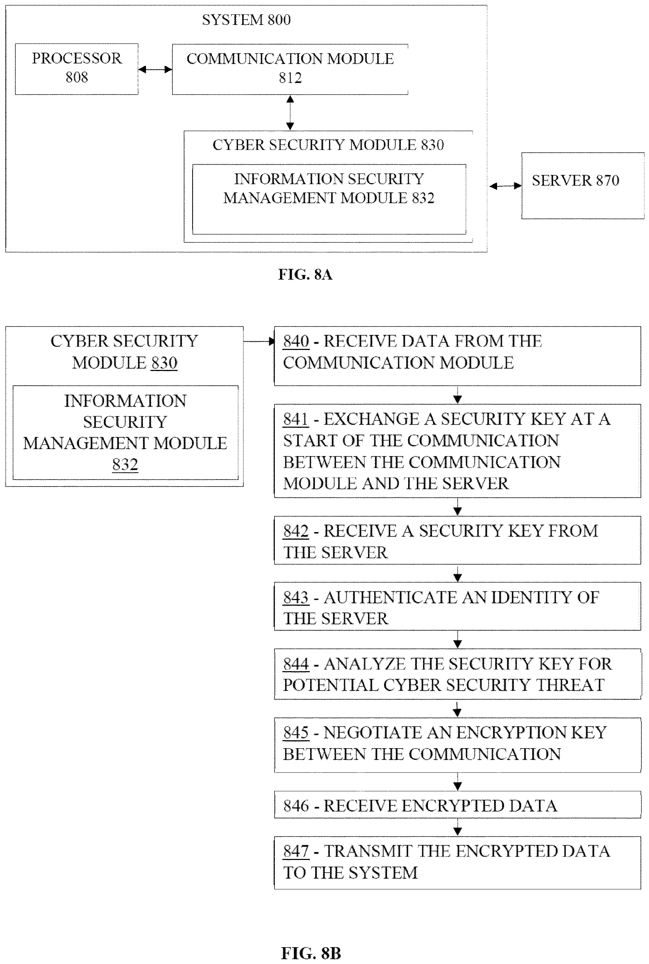

In an embodiment, FIG. 8A shows the block diagram of the cyber security module. The communication of data between the system 800 and the server 870 through the communication module 812 is first verified by the information security management module 832 before being transmitted from the system to the server or from the server to the system. The information security management module is operable to analyze the data for potential cyber security threats, to encrypt the data when no cyber security threat is detected, and to transmit the data encrypted to the system or the server.

In an embodiment, the cyber security module further comprises an information security management module providing isolation between the system and the server. FIG. 8B shows the flowchart of securing the data through the cyber security module 830. At step 840, the information security management module is operable to receive data from the communication module. At step 841, the information security management module exchanges a security key at a start of the communication between the communication module and the server. At step 842, the information security management module receives a security key from the server. At step 843, the information security management module authenticates an identity of the server by verifying the security key. At step 844, the information security management module analyzes the security key for potential cyber security threats. At step 845, the information security management module negotiates an encryption key between the communication module and the server. At step 846, the information security management module receives the encrypted data. At step 847, the information security management module transmits the encrypted data to the server when no cyber security threat is detected.

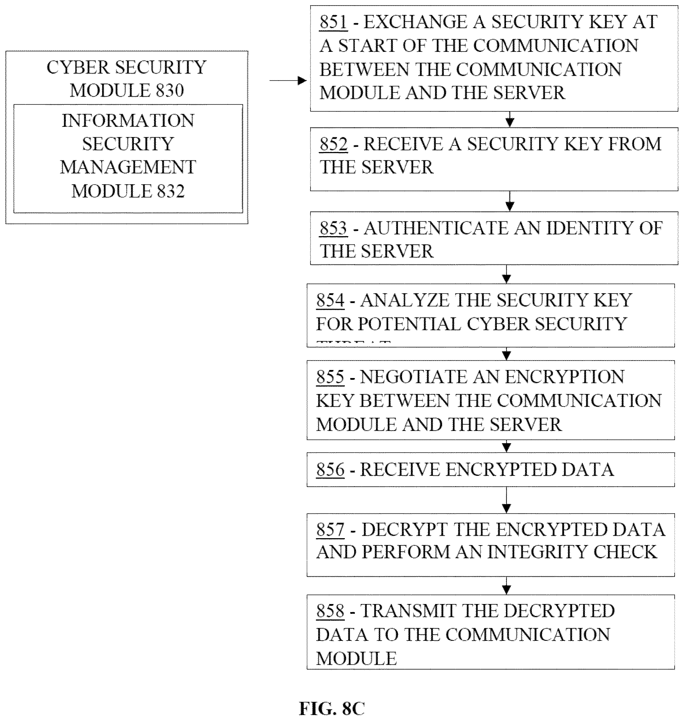

In an embodiment, FIG. 8C shows the flowchart of securing the data through the cyber security module 830. At step 851, the information security management module is operable to: exchange a security key at a start of the communication between the communication module and the server. At step 852, the information security management module receives a security key from the server. At step 853, the information security management module authenticates an identity of the server by verifying the security key. At step 854, the information security management module analyzes the security key for potential cyber security threats. At step 855, the information security management module negotiates an encryption key between the communication module and the server. At step 856, the information security management module receives encrypted data. At step 857, the information security management module decrypts the encrypted data, and performs an integrity check of the decrypted data. At step 858, the information security management module transmits the decrypted data to the communication module when no cyber security threat is detected.

In an embodiment, the integrity check is a hash-signature verification using a Secure Hash Algorithm 256 (SHA256) or a similar method.

In an embodiment, the information security management module is configured to perform asynchronous authentication and validation of the communication between the communication module and the server.

In an embodiment, the information security management module is configured to raise an alarm if a cyber security threat is detected. In an embodiment, the information security management module is configured to discard the encrypted data received if the integrity check of the encrypted data fails.

In an embodiment, the information security management module is configured to check the integrity of the decrypted data by checking accuracy, consistency, and any possible data loss during the communication through the communication module.

In an embodiment, the server is physically isolated from the system through the information security management module. When the system communicates with the server as shown in FIG. 8A, identity authentication is first carried out on the system and the server. The system is responsible for communicating/exchanging a public key of the system and a signature of the public key with the server. The public key of the system and the signature of the public key are sent to the information security management module. The information security management module decrypts the signature and verifies whether the decrypted public key is consistent with the received original public key or not. If the information security management module verifies decrypted public key, the identity authentication is passed. Similarly, the system and the server carry out identity authentication on the information security management module. After the identity authentication is passed on to the information security management module, the two communication parties, the system, and the server, negotiate an encryption key and an integrity check key for data communication of the two communication parties through the authenticated asymmetric key. A session ID number is transmitted in the identity authentication process, so that the key needs to be bound with the session ID number; when the system sends data to the outside, the information security gateway receives the data through the communication module, performs integrity authentication on the data, then encrypts the data through a negotiated secret key, and finally transmits the data to the server through the communication module. When the information security management module receives data through the communication module, the data is decrypted first, integrity verification is carried out on the data after decryption, and if verification is passed, the data is sent out through the communication module; otherwise, the data is discarded.

In an embodiment, the identity authentication is realized by adopting an asymmetric key with a signature.

In an embodiment, the signature is realized by a pair of asymmetric keys which are trusted by the information security management module and the system, wherein the private key is used for signing the identities of the two communication parties, and the public key is used for verifying that the identities of the two communication parties are signed. Signing identity comprises a public and a private key pair. In other words, signing identity is referred to as the common name of the certificates which are installed in the user's machine.

In an embodiment, both communication parties need to authenticate their own identities through a pair of asymmetric keys, and a task in charge of communication with the information security management module of the system is identified by a unique pair of asymmetric keys.

In an embodiment, the dynamic negotiation key is encrypted by adopting an Rivest-Shamir-Adleman (RSA) encryption algorithm. RSA is a public-key cryptosystem that is widely used for secure data transmission. The negotiated keys include a data encryption key and a data integrity check key.

In an embodiment, the data encryption method is a Triple Data Encryption Algorithm (3DES) encryption algorithm. The integrity check algorithm is a Hash-based Message Authentication Code (HMAC-MD5-128) algorithm. When data is output, the integrity check calculation is carried out on the data, the calculated Message Authentication Code (MAC) value is added with the header of the value data message, then the data (including the MAC of the header) is encrypted by using a 3DES algorithm, the header information of a security layer is added after the data is encrypted, and then the data is sent to the next layer for processing. In an embodiment the next layer refers to a transport layer in the Transmission Control Protocol / Internet Protocol (TCP/IP) model.

The information security management module ensures the safety, reliability, and confidentiality of the communication between the system and the server through the identity authentication when the communication between the two communication parties starts the data encryption and the data integrity authentication. The method is particularly suitable for an embedded platform which has less resources and is not connected with a Public Key Infrastructure (PKI) system and may ensure that the safety of the data on the server may not be compromised by a hacker attack under the condition of the Internet by ensuring the safety and reliability of the communication between the system and the server.

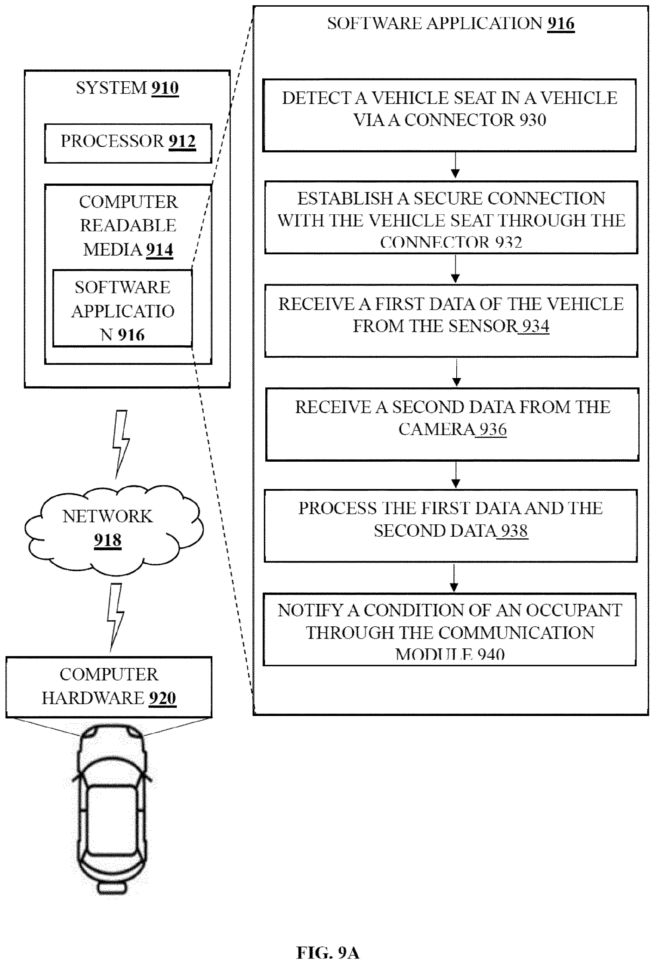

FIG. 9A shows the system with a software code to receiving alerts and health data of passengers according to an embodiment. The system 910 comprises a processor 912 and a computer readable media 914. The computer readable media 914 is a non-transitory storage media comprising a software application 916. The system 910 is in communication with computer hardware 920 inside the vehicle through a network 918 via a communication module in the system. The system 910 is configured for a software application 916 to be installed, via a software installation package provided over a computer network 918, onto the computing hardware 920 associated with the vehicle. The software application comprises a set of instructions executable by a computing hardware and stored in a non-transitory storage medium that, when executed, cause the computing hardware to implement operations comprising step described below:

In an embodiment, at step 930, the software application executes an instruction for detecting a vehicle seat in a vehicle via a connector. A handshaking signal is sent to the vehicle seat. The system receives an acknowledgement signal if the vehicle seat is connected to the system through the connector. Thus, the vehicle seat is detected.

At step 932, the software application executes an instruction for establishing a secure connection with the vehicle seat through the connector.

At step 934, the software application executes an instruction for receiving a first data of the vehicle from a sensor. The first data is a physiological condition of the occupant in the vehicle seat.

At step 936, the software application executes an instruction for receiving a second data from a camera. The camera captures the position of the occupant, size of the occupant according to the vehicle seat, seat belt position, conscious state of the occupant.

At step 938, the software application executes an instruction for processing the first data and the second data, by doing an analysis with a pre-set data of the occupant and detects whether there is a health emergency condition for the occupant or not.

At step 940, the software application executes an instruction for notifying a user a condition of the occupant through a communication module.

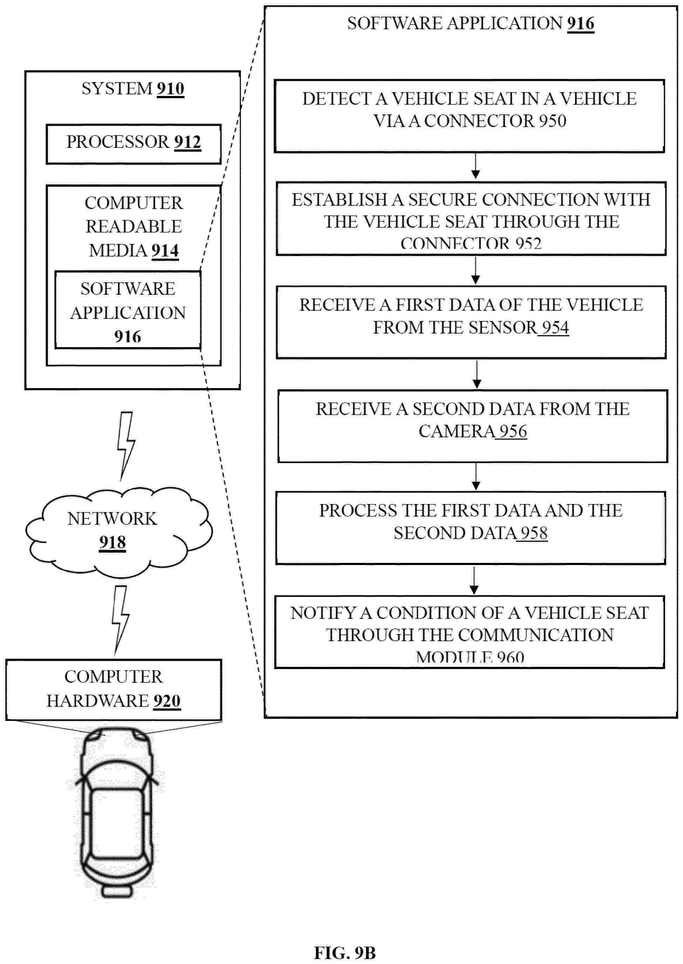

FIG. 9B shows the system with a software code to receiving alerts and condition of vehicle seat according to an embodiment. The system 910 comprises a processor 912 and a computer readable media 914. The computer readable media 914 is a non-transitory storage media comprising a software application 916. The system 910 is in communication with computer hardware 920 inside the vehicle through a network 918 via a communication module in the system. The system 910 is configured for installing a software application 916, via a software installation package provided over a computer network 918, onto the computing hardware 920 associated with the vehicle. The software application comprises a set of instructions executable by a computing hardware and stored in a non-transitory storage medium that, when executed, cause the computing hardware to implement operations comprising steps described herein:

In an embodiment, at step 950, the software application executes an instruction for detecting a vehicle seat in a vehicle via a connector. The system sends a handshaking signal to the vehicle seat. The system receives an acknowledgement signal if the vehicle seat is connected to the system through the connector. Thus, the vehicle seat is detected.

At step 952, the software application executes an instruction for establishing a secure connection with the vehicle seat through the connector.

At step 954, the software application executes an instruction for receiving a first data of the vehicle from a sensor. The first data of the vehicle seat further comprises data related to a vehicle seat position.

At step 956, the software application executes an instruction for receiving a second data from a camera. The camera captures the second data comprises a first image of the vehicle seat position and a second image of a size of the vehicle seat according to a height of an occupant.

At step 958, the software application executes an instruction for processing the first data and the second data, by doing an analysis of the data of the vehicle seat and detects whether the condition of the vehicle seat is good or not.

At step 960, the software application executes an instruction for notifying a user the condition of vehicle seat through a communication module.

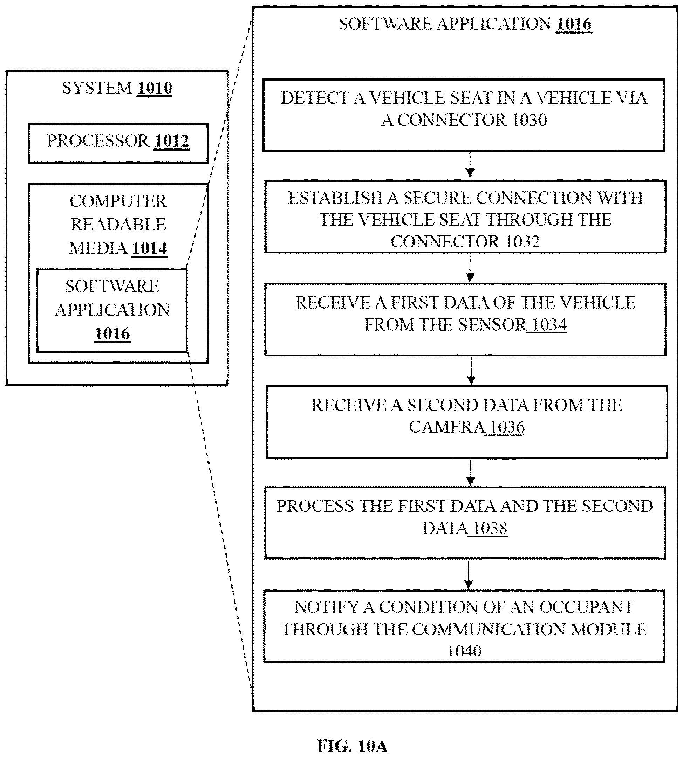

FIG. 10A shows the system with a non-transitory storage medium with the software code to receiving alerts and health data of passengers according to an embodiment. The system 1010 comprises a processor 1012 and a computer readable media 1014. The computer readable media 1014 is a non-transitory storage media comprising a software application 1016. The non-transitory medium executes the instructions as described herein:

In an embodiment, at step 1030, the software application executes an instruction for detecting a vehicle seat in a vehicle via a connector. The system sends a handshaking signal to the vehicle seat. The system receives an acknowledgement signal if the vehicle seat is connected to the system through the connector. Thus, the system detects the vehicle seat.

At step 1032, the software application executes an instruction for establishing a secure connection with the vehicle seat through the connector.

At step 1034, the software application executes an instruction for receiving a first data of the vehicle from a sensor. The first data is a physiological condition of the occupant in the vehicle seat.

At step 1036, the software application executes an instruction for receiving a second data from a camera. The camera captures the position of the occupant, size of the occupant according to the vehicle seat, seat belt position, and conscious state of the occupant.

At step 1038, the software application executes an instruction for processing the first data and the second data, by doing an analysis with a pre-set data of the occupant and detects whether there is a health emergency condition for the occupant.

At step 1040, the software application executes an instruction for notifying a user a condition of the occupant through a communication module.

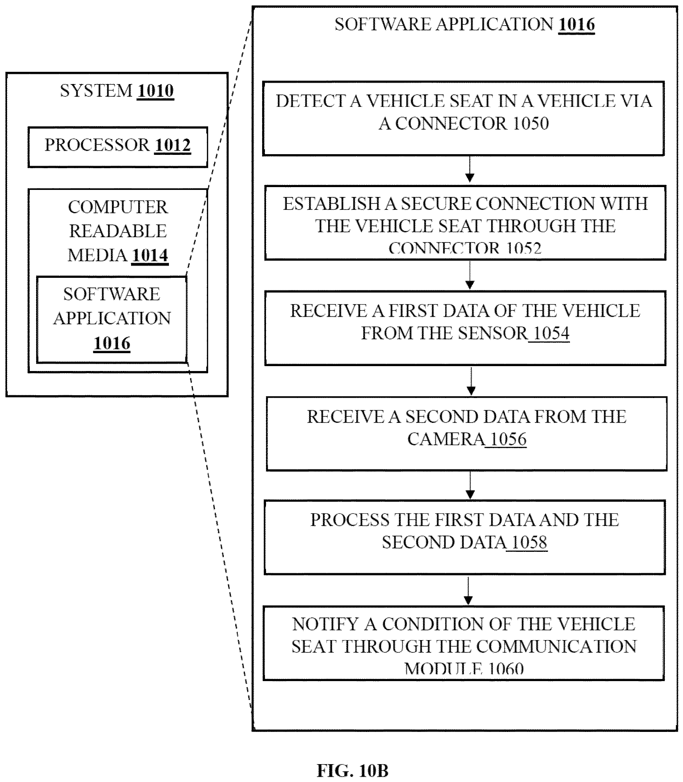

FIG. 10B shows the system with a non-transitory storage medium with the software application to receive alerts and condition of vehicle seat according to an embodiment. The system 1010 comprises a processor 1012 and a computer readable media 1014. The computer readable media 1014 is a non-transitory storage media comprising a software application 1016. The non-transitory medium executes the following instructions:

In an embodiment, at step 1050, the software application executes an instruction for detecting a vehicle seat in a vehicle via a connector. The system sends a handshaking signal to the vehicle seat. The system receives an acknowledgement signal if the vehicle seat is connected to the system through the connector. Thus, the system detects the vehicle seat.

At step 1052, the software application executes an instruction for establishing a secure connection with the vehicle seat through the connector.

At step 1054, the software application executes an instruction for receiving a first data of the vehicle from a sensor. The first data of the vehicle seat further comprises data related to a vehicle seat position.

At step 1056, the software application executes an instruction for receiving a second data from a camera. The camera captures the second data comprises a first image of the vehicle seat position and a second image of a size of the vehicle seat according to a height of an occupant.

At step 1058, the software application executes an instruction for processing the first data and the second data, by doing an analysis of the data of the vehicle seat and detects whether the condition of the vehicle seat is good.

At step 1060, the software application executes an instruction for notifying a user a condition of vehicle seat through a communication module.

The invention is also defined by the following clauses:
Clause 1. A system, wherein the system is configured to be a component of a vehicle; wherein the system comprising:
   a sensor;
   a camera;
   a connector;
   a communication module; and
   a processor; and
   wherein the processor is configured to:
      detect a vehicle seat in the vehicle via the connector;
      establish a secure connection with the vehicle seat through the connector;
      receive a first data of the vehicle from the sensor;
      receive a second data from the camera;
      process the first data and the second data; and
      notify a condition of the vehicle seat through the communication module.
Clause 2. The system of clause 1, wherein the vehicle seat comprises at least one of a normal car seat, a child car seat, and a pet car seat.
Clause 3. The system of clause 2, wherein the vehicle seat comprises at least one of a front facing car seat, rear facing car seat and a booster seat.
Clause 4. The system of clause 1, wherein the system comprises a plurality of sensors in the vehicle.
Clause 5. The system of clause 1, wherein the sensor comprises at least one of a seat belt sensor, a position sensor, a pressure sensor, a liquid detection sensor, weight sensor, an infrared sensor, an optical sensor, a comfort detection sensor, a moisture sensor, a temperature sensor, and an audio sensor.
Clause 6. The system of clause 1, wherein the camera comprises an infrared camera, and a thermal camera.
Clause 7. The system of clause 1, wherein the camera is rotatable at an angle to get a clear image.
Clause 8. The system of clause 1, wherein the system comprises a plurality of cameras.
Clause 9. The system of clause 1, wherein the first data of the vehicle seat further comprises data related to a vehicle seat position.
Clause 10. The system of clause 9, wherein the second data comprises a first image of the vehicle seat position and a second image of a size of the vehicle seat according to a height of an occupant.
Clause 11. The system of clause 10, wherein the first data comprises at least one of a first position of the vehicle seat, a second position of the occupant on the vehicle seat, a weight of the occupant, the height of the occupant, and a pressure on a seat belt.
Clause 12. The system of clause 1, wherein the connector comprises at least one of a wired and a wireless connector.
Clause 13. The system of clause 1, wherein the condition of the vehicle seat is displayed on a display.
Clause 14. The system of clause 13, wherein the condition of the vehicle seat is displayed on a vehicle infotainment system.
Clause 15. The system of clause 1, wherein the condition of the vehicle seat is displayed on a screen of a mobile device having an application.
Clause 16. The system of clause 1, wherein the processor is configured to:
   send a start signal to the vehicle seat through the connector; and
   receive an acknowledgement signal from the vehicle seat via the connector to detect the vehicle seat in the vehicle.
Clause 17. The system of clause 1, wherein the processor is configured to:
   receive a signal at the communication module from the vehicle seat;
   automatically determine if the signal originates from inside the vehicle;
   if the signal does not originate from inside the vehicle, reject the signal;
   if the signal originates from inside the vehicle, communicatively connect the communication module to the vehicle seat;
   receive a message from the vehicle seat; and
   verify that the message originated from inside the vehicle.
Clause 18. The system of clause 1, wherein the communication module is enabled for at least one of a vehicle-to-vehicle communication, vehicle-to-infrastructure communication, and vehicle-to-everything communication.
Clause 19. The system of clause 18, wherein the vehicle-to-vehicle communication comprises dedicated short-range communication.
Clause 20. The system of clause 1, wherein the processor is further configured to:
   perform a predictive diagnostic on the first data and the second data using artificial intelligence.
Clause 21. The system of clause 20, wherein the processor is further configured to:
   detect an emergency from the predictive diagnostic performed on the first data and the second data.
Clause 22. The system of clause 1, wherein the system further comprises cyber security module.
Clause 23. The system of clause 22, wherein the cyber security module further comprises an information security management module providing isolation between the system and a server.
Clause 24. The system of clause 23, wherein the information security management module is operable to:
   receive data from the communication module;
   exchange a security key at a start of a communication between the communication module and the server;
   receive the security key from the server;
   authenticate an identity of the server by verifying the security key;
   analyze the security key for a potential cyber security threat;
   negotiate an encryption key between the communication module and the server; encrypt the data; and
   transmit the encrypted data to the server when no cyber security threat is detected.
Clause 25. The system of clause 23, wherein the information security management module is operable to:
   exchange a security key at a start of a communication between the communication module and the server;
   receive the security key from the server;
   authenticate an identity of the server by verifying the security key;
   analyze the security key for a potential cyber security threat;
   negotiate an encryption key between the system and the server;
   receive encrypted data from the server;
   decrypt the encrypted data;
   perform an integrity check of the decrypted data; and
   transmit the decrypted data to the communication module when no cyber security threat is detected.
Clause 26. A method comprising:
   detecting a vehicle seat in a vehicle via a connector;
   establishing a secure connection with the vehicle seat through the connector;
   receiving a first data of the vehicle from a sensor;
   receiving a second data from a camera;
   processing the first data and the second data; and
   notifying a condition of the vehicle seat through a communication module.
Clause 27. A non-transitory computer storage medium storing a sequence of instructions, which when executed by a processor, causes:
   detecting a vehicle seat in a vehicle via a connector;
   establishing a secure connection with the vehicle seat through the connector;
   receiving a first data of the vehicle from a sensor;
   receiving a second data from a camera;
   processing the first data and the second data; and
   notifying a condition of the vehicle seat through a communication module.
Clause 28. A system, wherein the system is configured to be a component of a vehicle; wherein the system comprising:
   a sensor;
   a camera;
   a connector;
   a communication module; and
   a processor; and
   wherein the processor is configured to:
      detect a vehicle seat in the vehicle via the connector;
      establish a secure connection with the vehicle seat through the connector;
      receive a first data of the vehicle from the sensor;
      receive a second data from the camera;
      process the first data and the second data; and
      notify a condition of an occupant through the communication module.
Clause 29. The system of clause 28, wherein the vehicle seat comprises at least one of al car seat, a child car seat, and a pet car seat.
Clause 30. The system of clause 28, wherein the vehicle seat comprises at least one of a front facing car seat, rear facing car seat and a booster seat.
Clause 31. The system of clause 28, wherein the system comprises a plurality of sensor in the vehicle.
Clause 32. The system of clause 31, wherein the sensor comprises at least one of a seat belt sensor, a position sensor, a pressure sensor, a liquid detection sensor, weight sensor, an infrared sensor, an optical sensor, a comfort detection sensor, a moisture sensor, a temperature sensor, and an audio sensor.
Clause 33. The system of clause 28, wherein the camera comprises an infrared camera, and a thermal camera.
Clause 34. The system of clause 28, wherein the sensor comprises at least one of a heart rate sensor, a temperature sensor, a blood pressure sensor, a blood presence sensor, and a blood composition sensor.
Clause 35. The system of clause 28, wherein the second data comprises a first image of a vehicle seat position, a second image of a second position of the occupant on the vehicle seat, and a third image of a size of the vehicle seat according to a height and a weight of the occupant.
Clause 36. The system of clause 28, wherein the first data comprises at least one of a first position of the vehicle seat, a second position of the occupant on the vehicle seat, a weight of the occupant, a height of the occupant, and a pressure on a seat belt.
Clause 37. The system of clause 28 wherein the connector comprises at least one of a wired and a wireless connector.
Clause 38. The system of clause 28, wherein the condition of the occupant is displayed on a display.
Clause 39. The system of clause 38, wherein the condition of the occupant is displayed on a vehicle infotainment system.
Clause 40. The system of clause 28, wherein the condition of the occupant is displayed on a display of a mobile device having an application.
Clause 41. A method comprising:
   detecting a vehicle seat in a vehicle via a connector;
   establishing a secure connection with the vehicle seat through the connector;
   receiving a first data of the vehicle from a sensor;
   receiving a second data from a camera;
   processing the first data and the second data; and
   notifying a condition of an occupant through a communication module.
Clause 42. A non-transitory computer storage medium storing a sequence of instructions, which when executed by a processor, causes:
   detecting a vehicle seat in a vehicle via a connector;
   establishing a secure connection with the vehicle seat through the connector;
   receiving a first data of the vehicle from a sensor;
   receiving a second data from a camera;
   processing the first data and the second data; and
   notifying a condition of an occupant through a communication module.
Clause 43. A system, configured to:
   receive a software application installation package over a computer network; and
   install the software application onto a computing hardware associated with a vehicle;
   wherein the software application comprises:
      set of instructions executable by the computing hardware and stored in a non-transitory storage medium that, when executed, cause the computing hardware to implement operations comprising:
      detecting a vehicle seat in the vehicle via a connector;
      establishing a secure connection with the vehicle seat through the connector;
      receiving a first data of the vehicle from a sensor;
      receiving a second data from a camera;
      processing the first data and the second data; and
      notifying a condition of an occupant through a communication module.
Clause 44. A system, configured to:
   receive a software application installation package over a computer network; and
   install the software application onto a computing hardware associated with a vehicle;
   wherein the software application comprises:
      set of instructions executable by the computing hardware and stored in a non-transitory storage medium that, when executed, cause the computing hardware to implement operations comprising:
      detecting a vehicle seat in the vehicle via a connector;
      establishing a secure connection with the vehicle seat through the connector;
      receiving a first data of the vehicle from a sensor;
      receiving a second data from a camera;
      processing the first data and the second data; and
      notifying a condition of the vehicle seat through a communication module.
Clause 45. A system, wherein the system is configured to be a component of a vehicle; wherein the system comprising:
   a sensor;
   a camera;
   a connector; wherein a car seat connects to the vehicle via the connector;
   a communication module; and
   a processor; and
   wherein the processor is configured to:
      detect a car seat in the vehicle via the connector;
      establish a secure connection with the car seat through the connector;
      receiving health data from the car seat via the sensor and the camera;
      process the health data;
      detect a health issue of an occupant in the vehicle; and
      notify the health of an occupant through the communication module.

## Claims

1. A system (400), wherein the system is configured to be a component of a vehicle (401);
wherein the system (400) comprises:
a sensor (404) ;
a camera; (406)
a connector (410);
a communication module (420); and
a processor (402); and
wherein the processor is configured to:
detect a vehicle seat in the vehicle via the connector (421);
establish a secure connection with the vehicle seat through the connector (422);
receive a first data of the vehicle from the senso (424)r;
receive a second data from the camera (426);
process the first data and the second data (428) ;
notify a condition of the vehicle seat through the communication module (450); and
notify the condition of an occupant through the communication module (430).

2. The system (400) of claim 1 , wherein the vehicle seat comprises at least one of a normal car seat, a child car seat and a pet car seat.

3. The system (400) of any one of the preceding claims , wherein the system comprises a plurality of sensors (404) in the vehicle; and wherein the sensor comprises at least one of a seat belt sensor, a position sensor, a pressure sensor, a liquid detection sensor, weight sensor, an infrared sensor, an optical sensor, a comfort detection sensor, a moisture sensor, a temperature sensor, and an audio sensor.

4. The system (400) of any one of the preceding claims, wherein the camera (406) comprises an infrared camera, and a thermal camera; and/or
wherein the camera (406) is rotatable at an angle to get an image.

5. The system (400) of any one of the preceding claims, wherein the first data comprises at least one of a first position of the vehicle seat, a second position of the occupant on the vehicle seat, a weight of the occupant, a height of the occupant, and a pressure on a seat belt; and/or wherein the second data comprises a first image of a vehicle seat position, a second image of a second position of the occupant on the vehicle seat, and a third image of a size of the vehicle seat according to a height and a weight of the occupant.

6. The system (400) of any one of the preceding claims, wherein the sensor for monitoring a health data comprises at least one of a heart rate sensor, a temperature sensor, a blood pressure sensor, a blood presence sensor, and a blood composition sensor.

7. The system (400) of any one of the preceding claims, wherein the connector comprises at least one of a wired and a wireless connector.

8. The system (400) of any one of the preceding claims, wherein the condition of the vehicle seat and the condition of the occupant is displayed on a vehicle infotainment system and/or
on a screen of a mobile device having an application.

9. The system (400) of any one of the preceding claims, wherein the processor is configured to:
send a start signal to the vehicle seat through the connector; and
receive an acknowledgement signal from the vehicle seat via the connector to detect the vehicle seat in the vehicle; and/or
receive a signal at the communication module from the vehicle seat;
automatically determine if the signal originates from inside the vehicle;
if the signal does not originate from inside the vehicle, reject the signal;
if the signal originates from inside the vehicle, communicatively connect the communication module to the vehicle seat;
receive a message from the vehicle seat; and
verify that the message originated from inside the vehicle; and/orperform a predictive diagnostic on the first data and the second data using artificial intelligence and detect an emergency from the predictive diagnostic performed on the first data and the second data.

10. A method comprising:
detecting a vehicle seat in a vehicle via a connector (510);
establishing a secure connection with the vehicle seat through the connector (512);
receiving a first data of the vehicle from a sensor (514);
receiving a second data from a camera (516);
processing the first data and the second data (518);
notifying a condition of the vehicle seat through a communication module (540); and
notify the condition of an occupant through the communication module (520).

11. The method of claim 10, wherein the first data comprises at least one of a first position of the vehicle seat, a second position of the occupant on the vehicle seat, a weight of the occupant, a height of the occupant, and a pressure on a seat belt.

12. The method of claims 10 to 11, wherein the second data comprises a first image of a vehicle seat position, a second image of a second position of the occupant on the vehicle seat, and a third image of a size of the vehicle seat according to a height and a weight of the occupant.

13. The method of claims 10 to 12, wherein the method further comprises:
sending a start signal to the vehicle seat through the connector; and
receiving an acknowledgement signal from the vehicle seat via the connector to detect the vehicle seat in the vehicle.

14. The method of claims 10 to 13, wherein the method further comprises:
receiving a signal at the communication module from the vehicle seat;
automatically determining if the signal originates from inside the vehicle;
if the signal does not originate from inside the vehicle, rejecting the signal;
if the signal originates from inside the vehicle, communicatively connecting the communication module to the vehicle seat;
receiving a message from the vehicle seat; and
verifying that the message originated from inside the vehicle.

15. A non-transitory computer storage medium storing a sequence of instructions, which when executed by a processor carry out the method of any one of the claims 10 to 14.
